(19) **Europäisches Patentamt European Patent Office Office européen des brevets**

(11) **EP 1 470 410 B1**

(12) **EUROPEAN PATENT SPECIFICATION**

(45) Date of publication and mention
of the grant of the patent:
**11.01.2012 Bulletin 2012/02**

(51) Int Cl.:
***G01N 21/47*** (2006.01)  ***A61B 3/00*** (2006.01)
***G01B 9/02*** (2006.01)

(21) Application number: **03732098.3**

(22) Date of filing: **24.01.2003**

(86) International application number:
**PCT/US2003/002349**

(87) International publication number:
**WO 2003/062802 (31.07.2003 Gazette 2003/31)**

(54) **APPARATUS AND METHOD FOR RANGINGS AND NOISE REDUCTION OF LOW COHERENCE INTERFEROMETRY (LCI) AND OPTICAL COHERENCE TOMOGRAPHY (OCT) SIGNALS BY PARALLEL DETECTION OF SPECTRAL BANDS**

VORRICHTUNG UND VERFAHREN ZUR ORTUNG UND VERMINDERUNG DES RAUSCHENS VON SIGNALEN IN DER NIEDRIGKOHÄRENZINTERFEROMETRIE (LCI) UND DER OPTISCHE KOHÄRENZTOMOGRAFIE (OCT) MITTELS PARALLELDETEKTION VON SPEKTRALBÄNDERN

APPAREIL ET PROCEDE DE TELEMETRIE ET DE REDUCTION DU BRUIT POUR DES SIGNAUX D'INTERFEROMETRIE A FAIBLE COHERENCE (IFC) ET DE TOMOGRAPHIE A COHERENCE OPTIQUE (TCO) COMPRENANT LA DETECTION D'UN ENSEMBLE PARALLELE DE BANDES SPECTRALES

(84) Designated Contracting States:
**AT BE BG CH CY CZ DE DK EE ES FI FR GB GR HU IE IT LI LU MC NL PT SE SI SK TR**

(30) Priority: **24.01.2002 US 351904 P**
**30.04.2002 US 136813**

(43) Date of publication of application:
**27.10.2004 Bulletin 2004/44**

(73) Proprietor: **THE GENERAL HOSPITAL CORPORATION**
**Boston, MA 02114 (US)**

(72) Inventors:
- **DE BOER, Johannes, Fitzgerald**
  **Somerville, MA 02145 (US)**
- **TEARNEY, Guillermo, J.**
  **Cambridge, MA 02139 (US)**
- **BOUMA, Brett, Eugene**
  **Quincy, MA 02171 (US)**

(74) Representative: **Harris, Ian Richard et al**
**D Young & Co LLP**
**120 Holborn**
**London EC1N 2DY (GB)**

(56) References cited:
**WO-A-99/44089        GB-A- 2 209 221**
**US-A- 4 631 498        US-A- 4 925 302**
**US-A- 5 317 389        US-A- 5 321 501**
**US-A- 5 491 552        US-A- 6 069 698**
**US-A- 6 134 003        US-A- 6 141 577**

- **TEARNEY G J ET AL: "IN VIVO ENDOSCOPIC OPTICAL BIOPSY WITH OPTICAL COHERENCE TOMOGRAPHY" SCIENCE, AMERICAN ASSOCIATION FOR THE ADVANCEMENT OF SCIENCE,, US, vol. 276, no. 5321, 27 June 1997 (1997-06-27), pages 2037-2039, XP001041246 ISSN: 0036-8075**

**Description**

FIELD OF THE INVENTION

The present invention relates to apparatus, method, logic arrangement and storage medium for dramatically increasing the sensitivity in the detection of optical coherence tomography and low coherence interferometry signals by detecting a parallel set of spectral bands, each band being a unique combination of optical frequencies.

BACKGROUND OF THE ART

Two methods currently exist to implement depth ranging in turbid media. The first method is known as Low Coherence Interferometry ("LCI"). This method uses a scanning system to vary the reference arm length and acquire the interference signal at a detector and demodulating the fringe pattern to obtain the coherence envelope of the source cross correlation function. Optical coherence tomography ("OCT") is a means for obtaining a two-dimensional image using LCI. OCT is described by Swanson et al. in U.S. Pat. No. 5,321,501. Multiple variations on OCT have been patented, but many suffer from less than optimal signal to noise ratio ("SNR"), resulting in non-optimal resolution, low imaging frame rates, and poor depth of penetration. Power usage is a factor in such imaging techniques. For example in ophthalmic uses, only a certain number of milliwatts of power is tolerable before thermal damage can occur. Thus, boosting power is not feasible to increase SNR in such environments. It would be desirable to have a method of raising the SNR without appreciably increasing power requirements.

A second method for depth ranging in turbid media is known in the literature as spectral radar. In spectral radar the real part of the cross spectral density of sample and reference arm light is measured with a spectrometer. Depth profile information is encoded on the cross-spectral density modulation. Prior designs for spectral radar is primarily found in the literature.

The use of spectral radar concepts to increase the signal to noise ratio of LCI and OCT have been described earlier. However, in this description, only the real part of the complex spectral density is measured and the method uses a large number of detector elements (about 2,000) to reach scan ranges on the order of a millimeter. It would be desirable to have a method that would allow for an arbitrary number of detector elements. Secondly, the previously described method uses a single charge coupled device ("CCD"') to acquire the data. Since the charge storage capacity is limited, it requires a reduction of the reference arm power to approximately the same level as the sample arm power, giving rise to auto correlation noise on the sample arm light. In addition, since no carrier is generated, the I/f noise will dominate the noise in this system. Thirdly, even with the short integration times of state of the art CCD technology, phase instabilities in the interferometer reduce fringe visibility of the cross spectral density modulation.

In US6,134,003, a system for performing optical coherence tomography is described which views different tissue depths by moving a reference arm.

In US5,317,389, an apparatus for optical imaging is described which comprises:

1) a low coherence light source;
2) an interferometer;
3) a spectral separating unit which splits a signal received from the inferometer into a plurality of spectral bands;
4) a plurality of detectors, each detector being arranged to detect at least a portion of the spectral bands and to generate second signals as a function of the detected spectral bands; and
5) a signal processing arrangement which is configured to process the second signals to generate a surface topology of a sample.

SUMMARY OF THE INVENTION

The present invention can increase the SNR of LCI and OCT by splitting the LCI broad bandwidth source into a number "N" of spectral bands. In one exemplary embodiment, the N spectral bands are individually detected and processed to provide an increase in the SNR by a factor of N. This increase in SNR enables LCI or OCT imaging by a factor of N times faster, or alternatively allows imaging at the same speed with a source that has N times lower power. As a result, the present invention overcomes two of the most important shortcomings of conventional LCI and OCT, namely, source availability and scan speed. The factor N may reach more than 1,000, and allows construction of OCT and LC1 systems that can be more than three orders of magnitude improved from OCT and LCI technology currently in practice.

The present invention improves current data acquisition speeds and availability of sources for OCT. Shot noise is due to the statistical fluctuations of the current that are due to the quantized or discrete electric charges. The reduction of shot noise allows for much lower source powers or much higher acquisition rates. Limitations in current data acquisition rates (approximately 4 frames/sec) are imposed by available source power and availability of fast mechanisms for scanning delay. An increase in the sensitivity of the detection by a factor of 8 would allow real time imaging at a speed of about 30 frames per second. An increase of the sensitivity by a factor of about 1,000-2,000 would allow for the use of sources with much lower powers and higher spectral bandwidths which are readily available, cheaper to produce, and can generate higher resolution LCI or OCT scans.

For ophthalmic applications of OCT, the efficient detection preferably allows for a significant increase of acquisition speed. The limitation in ophthalmic applications is the power that is allowed to enter the eye according to the ANSI standards (approximately 700 microwatts at 830 nm). Current data acquisition speed in ophthalmic applications is approximately 100-500 A-lines per second. The power efficient detection would allow for A-line acquisition rates on the order of about 100,000 A-lines per second, or video rate imaging at about 3,000 A-lines per image.

The gain in SNR is achieved because the shot noise has a white noise spectrum. An intensity present at the detector at frequency $\omega$(or wavelength $\lambda$) contributes only to the signal at frequency $\omega$, but the shot noise is generated at all frequencies. By narrowing the optical band width per detector, the shot noise contribution at each frequency can be reduced, while the signal component remains the same.

In summary, the present invention improves a performance of LCI and OCT, and as a result, can be used in developing LCI and OCT diagnostic technologies for medical and non-medical applications.

Other features and advantages of the present invention will become apparent upon reading the following detailed description of embodiments of the invention, when taken in conjunction with the appended claims.

BRIEF DESCRIPTION OF THE DRAWINGS

The invention is illustrated in the drawings in which like reference characters designate the same or similar parts throughout the figures of which:

Fig. 1 is a schematic view of a conventional system.

Fig. 2 is a schematic view of a preferred embodiment of the parallel detection scheme for LCI.

Fig. 3 is a schematic view of a system with one detector array according to one embodiment of the present invention.

Fig. 4 is a detail of a probe.

Fig. 5 is a schematic view of separating unit in combination with two integrating CCD arrays for detection of the dual-balanced wavelength demultiplexed signal.

Fig. 6 is a schematic view of a preferred embodiment of a standalone system

Fig. 7 is a schematic view showing spectral separating into 2 bands.

Fig. 8 is a schematic of spectral separating into 4 bands. The spectral resolution preferably used for each detector is twice as coarse as in the case of multiplexing into 2 bands.

Fig. 9 is a schematic view of using beam recombination to provide one dimension of interference information along one dimension of a two-dimensional detector array, while performing wavelength separating along the other dimension of the two dimensional array.

Fig. 10 is a schematic view of a phase tracking system according to one embodiment of the present invention.

Fig. 11 is a flowchart depicting the reconstruction of LCI or OCT signal from wavelength bands.

Fig. 12 is a schematic view of a spectral domain OCT interferometer design with a source combining the spectra of several superluminescent sources.

Fig. 13 is a schematic view of a system with a four detector array.

Fig. 14 is a graph of a typical interference patter as a function of path length difference between sample arm and reference arm.

Fig. 15 is an embodiment of a phase tracker system with an extended phase lock range.

Figs. 15A-C are flow diagrams of a method.

Fig. 16 is a graph of frequency versus OCT power spectrum.

Fig. 17 is a graph of frequency versus amplitude spectrum subtracted from the shot noise (experimental data) for the N=1 (dotted line) and N=1/3 (solid line) cases.

Fig. 18 is a graph of power density for the full spectrum as a function of frequency.

Fig. 19 is a graph after subtraction of the shot noise levels.

Fig. 20 is a graph after processing the signals.

Fig. 21 is a graph of the coherence envelope for the coherently summed channels.

## DETAILED DESCRIPTION OF THE PREFERRED EMBODIMENTS

### Overview

Certain exemplary embodiments of the present invention include a hybrid method that implements aspects of LCI and OCT where the reference arm is scanned, and spectral radar, which does not require reference arm scanning.

In one embodiment, the signal in the detection arm of an OCT system is split into more than one spectral band before detection. Each spectral band is detected by a separate photodetector and amplified. For each spectral band, the signal can be band pass filtered around the signal band by analog electronics and digitized, or, alternatively, the signal may be digitized and band pass filtered in software. As a consequence, the shot noise contribution to the signal can be reduced by a factor equal to the number of spectral bands, while output of the signal remains the same. The reduction of the shot noise increases the dynamic range and sensitivity of the system.

An apparatus for spectral radar that does not require reference arm scanning is described. For many detectors, no ranging or reference arm scanning is needed, and the method may be similar to the method which can be employed for a spectral radar except that phase information of the cross spectral density is preferably preserved.

Arrangement for spectral radar is described that eliminates phase instability in the interferometer, obtains the complex spectral density and eliminates auto correlation noise on the sample arm light, relative intensity noise, and 1/f noise.

### THEORY

#### TIME DOMAIN VERSUS SPECTRAL DOMAIN OCT

Nearly all conventional OCT systems are based on Time Domain scanning. In such conventional systems, the length of the reference arm in a Michelson interferometer is rapidly scanned over a distance corresponding to the imaging depth range. An alternative procedure to scanning the reference arm, is one that measures the cross-spectral density at the detection arm of the Michelson interferometer using a spectrometer. In Spectral Domain OCT, no mechanical (e.g., motionless) scanning of the reference arm is required, while an apparatus for generating a phase shift can be used. Only recently was it recognized that a significant signal to noise gain can be achieved by direct measurement of the cross-spectral density.

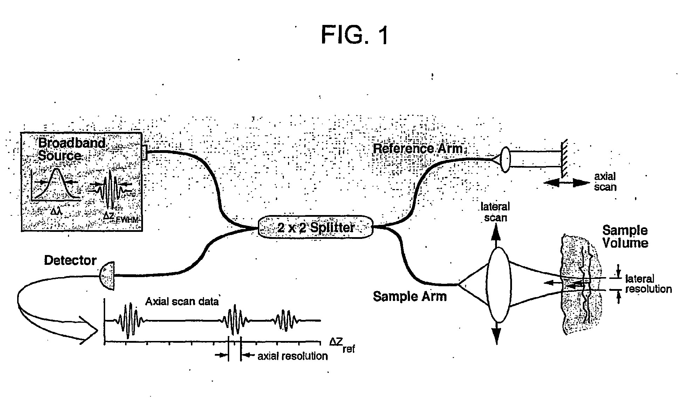

Fig. 1 shows a schematic of a conventional Time Domain OCT system. On scanning the reference arm path length, interference fringes are formed corresponding to positions that match the distance to the three structures drawn in the sample volume. A single detector is used to detect the interference fringes. By envelope detection of the fringe patterns, an image is constructed that maps tissue reflectivity to a given location.

Certain exemplary embodiments of the present invention provide a detection principle based on Spectral Radar concepts (further referred to as Spectral Domain OCT) or a hybrid method between Spectral Domain and Time Domain OCT that can be more sensitive than current state of the art Time Domain OCT, allowing a substantial increase in the acquisition speed to resolution ratio.

Principle of shot noise reduction in Spectral Domain OCT

The best signal to noise performance of Time Domain OCT systems is obtained when the noise is shot noise limited. Shot noise can be reduced significantly by replacing the single element detector with a multi-element array detector. When the detection arm light is spectrally dispersed on the array detector, each element of the array detects a small wavelength fraction of the spectral width of the source. The shot noise is preferably reduced by a factor equal to the number of elements of the array. The principle of the signal to noise improvement is based on the white noise characteristic of shot noise and the observation that only electromagnetic waves of the same wavelength produce interference fringes.

The shot noise power density $N_{shot}(f)$ (in units [$W/Hz$],[$A^2/Hz$] or [$V^2/Hz$]) is proportional to the current (or equivalently the optical power times the quantum efficiency) generated in the detector. For a monochromatic beam of wavelength $\lambda_1$ entering the interferometer, the fringe frequency or carrier $f$ at the detector is determined by the velocity v of the mirror, $f_1 = 2v/\lambda_1$. The shot noise is proportional to the power (or spectral density $S(\omega)$) at wavelength $\lambda_1$. A second wavelength $\lambda_2$ is preferably coupled into the interferometer. A second fringe frequency or carrier at frequency $f_2 = 2v/\lambda_2$ is simultaneously present. The shot noise at this second frequency is preferably the sum of the shot noise generated by the optical power at wavelength $\lambda_1$ and $\lambda_2$. Also, at frequency $f_1$ the shot noise is the sum of the shot noise generated by the optical power at wavelength $\lambda_1$ and $\lambda_2$. Thus, at both frequencies a cross-shot noise term is generated by the simultaneous presence of both wavelengths at the detector. By spectrally dispersing each wavelength to a separate detector, the cross shot noise term can be eliminated. In this way, Spectral Domain OCT offers a significant improvement of signal to noise ratio over Time Domain OCT systems.

Signal to noise analysis of Time Domain versus Spectral Domain OCT.

Signal

Analysis of the Signal to Noise Ratio (SNR) in Time Domain OCT has been described in related publications. The interference fringe peak amplitude in time domain OCT is given by

$$I_{peak} = \sqrt{P_{ref}P_{sample}} \, , \qquad (1)$$

with $P_{ref}$, $P_{sample}$ the reference and sample arm power in Watts, respectively. In terms of electrical power at the detector, the signal in units [$A^2$] is defined as

$$S = \eta^2 e^2 P_{ref}P_{sample}/E_v^2 \, , \qquad (2)$$

with $\eta$ the quantum efficiency, e the charge quantum and $E_v = hc/\lambda$ the photon energy. The reference and sample arm powers are given by the respective reflected spectral densities,

$$P_{ref,sample} = \int S_{ref,sample}(\omega)d\omega \, . \qquad (3)$$

Assuming that the reference and sample spectral densities are equal to the source spectral density $S(\omega)$, where the sample arm spectral density is attenuated by a large factor, i.e., $S_{ref}(\omega)= S(\omega)$, $S_{sample}(\omega) =\alpha\, S(\omega)$ with $\alpha \ll 1$, and inserting the above expression of reference and sample arm into the original definition of the signal gives,

$$S = \eta^2 e^2 \alpha \left[\int S(\omega)d\omega\right]^2 /E_v^2 . \qquad (4)$$

Thermal, shot noise and relative intensity noise contributions

Three contributions to the total noise of OCT signals are: thermal noise, shot noise and relative intensity noise. Thermal noise is generated by the feedback resistor, shot noise is related to the finite nature of the charge quantum resulting in statistical fluctuations on the current, and relative intensity noise is related to the temporal fluctuations due to chaotic character of classical light sources. These three contributions to the noise density in units [$A^2/Hz$] are given by,

$$N_{noise}(f) = \frac{4kT}{R_{fb}} + \frac{2\eta e^2 P_{ref}}{E_\nu} + 2\left(\frac{\eta e P_{ref}}{E_\nu}\right)^2 \tau_{coh}, \qquad (5)$$

$k$ is Boltzmann's constant, $T$ the temperature in Kelvin, $R_{fb}$ the value of the feedback resistor, and $\tau_{coh}$ the coherence time of the source. Coherence time is related to the full spectral width at half maximum $\Delta\lambda$ of a Gaussian source by the following relation, $\tau_{coh} = \sqrt{2\ln 2/\pi}\ \lambda_0^2/(c\,\Delta\lambda)$. Shot noise limited detection is achieved when the second term in Eq. (5) dominates the other noise contributions.

Signal to Noise Ratio (SNR)

The signal to noise ratio (SNR) is given by

$$SNR = \frac{S}{N_{noise}(f)\,BW}, \qquad (6)$$

with $BW$ the signal bandwidth, and parameters $S$ and $N_{noise}(f)$ as described above.

Space and frequency domain description of the OCT signal

The OCT signal is most easily described in the space domain. For a single object in the sample arm, the interference term of the OCT signal is proportional to the real part of the Fourier transform of the source spectrum $S(\omega)$,

$$I(\Delta z) \propto \mathrm{Re}\int \exp(ik\Delta z)S(k)\,dk, \qquad (7)$$

with $\Delta z$ the path length difference between sample and reference arm and k the wave vector. As a function of time, the OCT signal is given by,

$$I(t) \propto \mathrm{Re}\int \exp(2i\omega tv/c)S(\omega)\,d\omega, \qquad (8)$$

with v the reference arm mirror velocity. The frequency spectrum of the signal is given by a Fourier transform of the signal in the time domain, resulting in a complex function. The absolute value of this function is equal to the spectral density,

$$|I(f)| = \left|\int I(t)e^{2i\pi ft}dt\right| = S(\pi fc/v), \qquad (9)$$

which shows that the signal bandwidth is directly proportional to the source spectral width and scales linearly with the reference arm mirror velocity, i.e., imaging speed. Eq. (9) also directly relates the absolute value of the frequency spectrum, $|I(f)|$, to the signal S (Eq. (4)).

Eq (9) also demonstrates that each angular frequency of the light source or equivalently each wavelength of the source is represented at its own frequency in the measured interferometric signal. The depth profile information I(t) can be obtained from the complex cross spectral density $I(f)$ by a Fourier transform.

The complex cross spectral density can also be obtained by splitting the signal I(t) in several spectral bands using a dispersive or interferometric element. At each detector, only part of the complex cross spectral density is determined. Combining the cross spectral densities of each detector, the full spectral density of the signal is retrieved.

Thus, the same information can be obtained by separating spectral components to individual detectors. Combining the signal of all detectors in software or hardware would result in the same signal as obtained with a single detector.

Signal to noise gain with Spectral domain OCT

In the detection arm, the spectrum can be split into two equal halves, where two detectors each detect one half of the spectrum. According to Eq (9), the frequency spectra at detectors 1 and 2 are given by $|I_1(f)| = S(\pi fc/v)$ for $f < f_0$, $I_1(f) = 0$ for $f > f_0$ and $I_2(f) = 0$ for $f < f_0$, $|I_2(f)| = S(\pi fc/v)$ for $f > f_0$, respectively. The frequency spectrum as would be acquired by a single detector in time domain OCT is given by the sum of $I_1(f)$ and $I_2(f)$; $I(f) = I_1(f) + I_2(f)$. Thus, the signal S after combining the spectra is equal, however $I_1(f) = 0$ for $f > f_0$ and $I_2(f) = 0$ for $f < f_0$, the bandwidth BW per detector can be reduced by a factor of 2.

The noise is determined by the sum of the shot noise contributions at detectors one and two. From Eqs. (5) and (6), the shot noise per detector is proportional to the reference arm power at the detector times the bandwidth for the detector. Since the spectrum was split in equal halves, the reference power at detectors 1 and 2 is, respectively,

$$P_{ref}^1 = 0.5 P_{ref}, \quad P_{ref}^2 = 0.5 P_{ref}. \qquad (10)$$

The sum of the shot noise contribution for the two detectors is,

$$N_{noise}^{SD} \propto P_{ref}^1 \times 0.5 BW + P_{ref}^2 \times 0.5 BW = 0.5 P_{ref} BW, \qquad (11)$$

which may compared with the shot noise of a single detector in time domain OCT,

$$N_{noise}^{TD} \propto P_{ref} BW. \qquad (12)$$

Thus, by spectrally dispersing the detection arm light over two separate detectors, the signal remains the same, while the noise is reduced by a factor of 2, resulting in a net SNR gain by a factor of 2.

Extending the above analysis, it can be demonstrated that the shot noise contribution is reduced by a factor equal to the number of detectors. The sum of shot noises for N detector elements, where each detector element receives one Nth of the total reference power, is given by,

$$N_{noise} = \frac{2\eta e^2 P_{ref}}{E_v} \frac{BW}{N}. \qquad (13)$$

The signal is the same as in Time Domain OCT, and the SNR ratio for Spectral Domain OCT is given by,

$$\frac{S}{N_{noise}} = \frac{\eta P_{sample} N}{2 E_v BW}. \qquad (14)$$

Thus Spectral Domain OCT enables a SNR improvement over Time Domain OCT of a hundred to a thousand fold, depending on the number of detector elements N. Using a charge coupled array or an integrating device as a detector, such as, but not limited to, a line scan camera, the ratio N/BW is replaced by the integration time $\tau_i$ of the array, which results in,

$$\frac{S}{N_{noise}} = \frac{\eta P_{sample} \, \tau_i}{2E_v}.$$  (15)

<u>Advantages</u>

The exemplary embodiment of the present invention reduce shot noise and other forms of noise which allows for much lower source powers, or much higher acquisition rates than current systems. The increased detection sensitivity allows for real time imaging. Such imaging speed can help practitioners where motion artifacts are a continuing problem, such as in gastrointestinal, ophthalmic and arterial imaging environments. By increasing the frame rate while maintaining or improving the signal to noise ratio such artifacts can be minimized. The present invention also enable one to screen large areas of tissues with OCT and allows clinical viable screening protocols using this method.

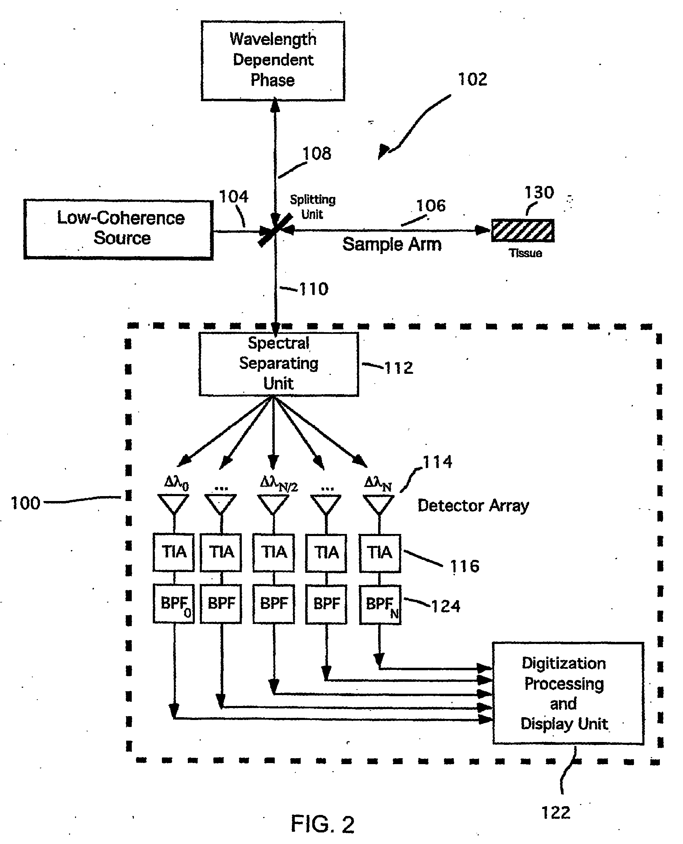

Fig. 2 shows a top level system 100 configuration according to an exemplary embodiment of the present invention, which comprises an interferometer 102 with a source arm 104, a sample arm 106, a reference arm 108, and a detection arm 110 with a spectral separating unit 112, multiple detectors 114, amplifiers 116, optional analog processing electronics 118 (not shown, but known to those skilled in the art), and A/D converters 120 (not shown, but known to those skilled in the art) for conversion of signals. A processing and display unit 122 has optionally digital band pass filtering ("BPF") units 124, Digital Fast Fourier Transforms ("FFTs") 126 (not shown), coherent combination of signals, and data processing and display algorithms. The detector array 114 may be 1xN for simple intensity ranging and imaging and/or Doppler sensitive detection, 2xN for dual balanced detection, 2xN for simple intensity ranging and/or polarization and/or Doppler sensitive detection, or 4xN for combined dual balanced and polarization and/or Doppler sensitive detection. Alternatively, an MxN array may be used for arbitrary number "M" of detectors 114 to allow detection of transverse spatial information on the sample 130.

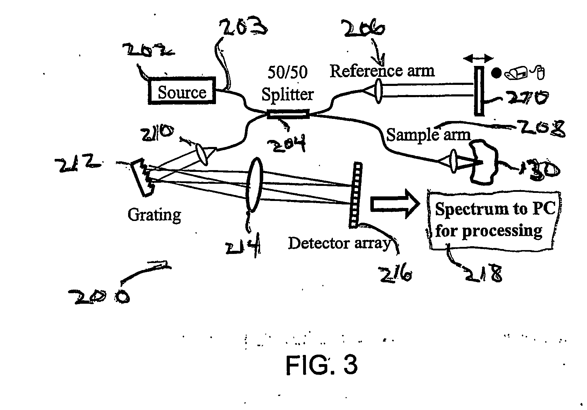

Fig. 3 shows a schematic of one exemplary embodiment of a Spectral Domain OCT system 200, which includes a light source 202, splitter 204, reference arm 206, sample arm 208, tissue sample 130, optical element 210, grating 212, lens 214, detector 216 array, and processor 218. The detection arm light is dispersed by the grating 212 and the spectrum imaged onto a detector array 216. By stepping the reference arm 206 length over a distance $\lambda/8$, the cross spectral density of reference arm 206 and sample arm 208 light can be determined. A Fourier transform of the cross spectral density generates the depth profile information.

<u>Sources</u>

The source arm 203 contains at least light source 202 that is used to illuminate the interferometer with low-coherence light. The source temporal coherence length is preferably shorter than a few microns (a preferred range is about 0.5 $\mu$m - 30 $\mu$m). Examples of sources include, but are not limited to, semiconductor optical amplifier, superluminescent diodes, light-emitting diodes, solid-state femtosecond sources, amplified spontaneous emission, continuum sources, thermal sources, combinations thereof and the like. Other appropriate sources known to those skilled in the art may be used. While light is referred to herein as the source, it is intended that other electromagnetic radiation ranges may be suitable for use, depending on the circumstances.

<u>Interferometer</u>

The sample arm 208 collects light reflected from the tissue sample 130 and is combined with the light from the reference arm 206 to form interference fringes. The reference arm 206 returns light back to be combined with the source arm 203. The reference arm can also be transmissive with no reflection. This action of beam splitting/recombining may be performed using a beam splitter 204 (Michelson), or circulator(s) (Mach-Zehnder) or other means known to those skilled in the art for separating a beam into multiple paths and recombining these multiple beams in a manner that interference between the beams may be detected. The splitting may be accomplished in free space or by using a splitter 204 having passive fiber optic or waveguide components.

Sample arm

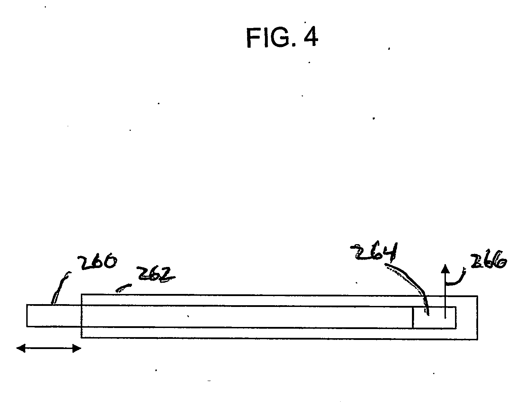

For LCI applications, the sample arm may be terminated by an optical probe comprising a cleaved (angled, flat, or polished) optical fiber or free space beam. A lens (such as, but not limited to, aspherical, gradient index, spherical, diffractive, ball, drum or the like) may be used to focus the beam on or within the sample. Beam directing elements (such as, but not limited to, mirror, prism, diffractive optical element or the like) may also be contained within the probe to direct the focused beam to a desired position on the sample. For OCT applications, the position of the beam may be changed on the sample as a function of time, allowing reconstruction of a two-dimensional image. Altering the position of the focused beam on the sample may be accomplished by a scanning mirror (such as, but not limited to, a galvanometer, piezoelectric actuator or the like), electrooptic actuator, or moving the optical fiber (for example, rotating the optical fiber, or linearly translating the optical fiber). The sample arm probe may be a fiber optic probe that has an internally moving element where the motion is initiated at a proximal end of the probe and the motion is conveyed by a motion transducing arrangement (such as, but not limited to, wire, guidewire, speedometer cable, spring, optical fiber and the like) to the distal end. The fiber optic probe may be enclosed in a stationary sheath which is optically transparent where the light exits the probe at the distal end. Fig. 4 shows a detail view having an inner cable 260 (which may rotate or linearly translate along the axis of the probe), an outer transparent or semi-transparent sheath 262, distal optics 264, and remitted light 266 (which may be at any angle with respect to axis of catheter).

Reference arm delay

A mechanism 270 in the reference arm 206 allows for scanning the group delay of the reference arm 206. This group delay can be produced by any of a number of techniques known to those skilled in the art, such as, but not limited to, stretching an optical fiber, free space translational scanning using a piezoelectric transducer, or via a grating based pulse shaping optical delay line. Preferably, the delay is introduced by a non-mechanical or motionless arrangement. By "non-mechanical" it is meant that no mechanically moving parts are utilized. The absence of mechanically moving parts is believed to reduce the known deficiencies of using mechanical devices to introduce delay. As opposed to traditional LCI or OCT systems described in the literature, the reference arm 206 in the present invention does not necessarily need to scan over the full ranging depth in the sample, and preferably scans over at least a fraction of the ranging depth equal to one over the number of detectors (1/N). This scanning feature is fundamentally different from known delay scanning schemes used in conventional known LCI and OCT systems. The reference arm 206 optionally has a phase modulator mechanism (described more fully herein), such as, but not limited to, an acoustooptic modulator, electrooptic phase modulator or the like, for generating a carrier frequency. In order to reduce the scan range of the reference arm 206, the spectrum is preferably split into a plurality of spectral bands according to a method that will be explained below.

Detection

Referring to Fig. 2, in the detection arm 110 spectral separating unit separates the spectral components and the signal is forwarded to separate detectors 114. The detectors 114 may preferably consist of photodiodes (such as, but not limited to, silicon, InGaAs, extended InGaAs, and the like). Alternatively, a one or two dimensional array of detectors 114 (such as, but not limited to, photodiode array, CCD, CMOS array, active CMOS array, CMOS "smart pixel" arrays, combinations thereof and the like) may be employed for detection. Two detectors 114 for each spectral band may be used for polarization sensitive detection following separation of the recombined light into orthogonal polarization eigenstates. Detector 114 arrays may be 1xN for simple intensity ranging and imaging and/or Doppler sensitive detection, 2xN for dual balanced detection, 2xN for intensity ranging and imaging and/or polarization sensitive and/or Doppler sensitive detection, or 4xN for combined dual balanced and intensity ranging and/or Doppler sensitive and/or polarization sensitive detection. Alternatively, an MxN array may be used for arbitrary M to allow detection of transverse spatial information on the sample 40.

Detector signals can be amplified by Trans Impedance Amplifiers ("TIAs") 116, band pass filters 124 (digitally or using analog circuitry) and digitized by A/D converters and stored in a computer 122 for further processing. Each detector 114 is preferably configured to be shot noise limited. Shot noise limited detection is preferably achieved by adjusting the intensity of light returned from the reference arm 108 so that the shot noise dominates over the thermal noise of the resistor in the TIA 116 and is higher than the relative intensity noise ("RIN"). Each detector 114 is balanced for such dual noise reduction.

In one embodiment of the present invention, the number of detectors 114, N can be in the range of 2-10,000 or more. A preferred range of N is about 8-10,000 detectors. In one preferred embodiment, eight detectors 114 (or a number in that area) can provide real time, or close to real time, imaging.

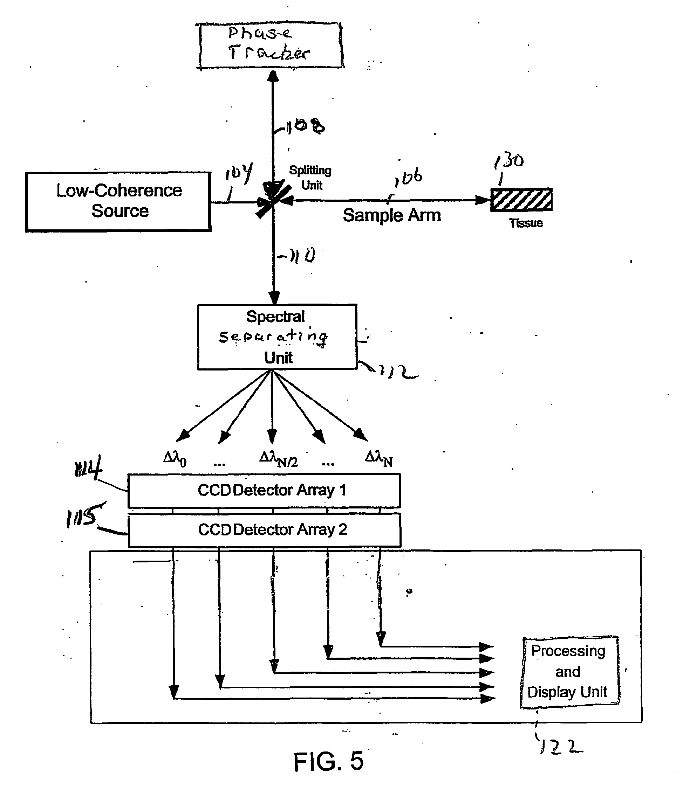

Alternatively, another way for detection includes an integrating one-dimensional or two-dimensional detector 114 array which is capable of obtaining images at a rate preferably greater than 1/f noise (f = frequency) (see Fig. 5). Optionally, the BPF can be implemented discretely following digitization. An additional modification includes using an optional second detector 115 array for balanced detection which allows increased reference arm power and acquisition speed due to reduction of RIN and 1/f noise. In a preferred embodiment, a phase tracking apparatus and/or algorithm is used in the reference arm 108 to reduce signal attenuation due to fringe instability.

This system could be implemented using a single detector 114 with dual-balanced detection enabled by either interleaving dual balanced rows of the array detector or by placing two similar array detectors adjacent to one another. If two array detectors 114 and 115 are used, the values are subtracted from one another to achieve dual balance detection. If more than two array detectors are used the signals can be selectively subtracted and complex spectral density can be obtained.

The spectral intensity as a function of wavelength is preferably constant. However, if it is not, the spectrum can be shaped in the reference, sample and/or source arms to make it constant. Spectral shapers are known in the art.

Processing

The signal of each detector 114 is band pass filtered around the signal frequency, such as by FFT's. The signal of all detectors 114 can be combined as explained hereinabove to obtain the complex cross spectral density in the frequency domain. By Fourier transform, the complex cross spectral density can be converted to a depth profile in the tissue. Several methods to process the complex spectral density to obtain depth profile information are known to those skilled in the art, such as, but not limited to, by obtaining at least two signals with a pi/2 phase shift in the reference arm and then reconstructing the complex spectral density by some linear combination of the two signals.

Following detection analog processing includes a trans impedance amplifier, band pass filter, and digitization of the signal. This signal may then be converted to reflectivity as a function of depth by the Fourier transform operation. Digital processing includes digitization, digital band pass filtering in either the frequency domain or time domain (FIR or IIR filter) and inverse Fourier transformation to recover the tissue reflectivity as a function of depth.

System Integration

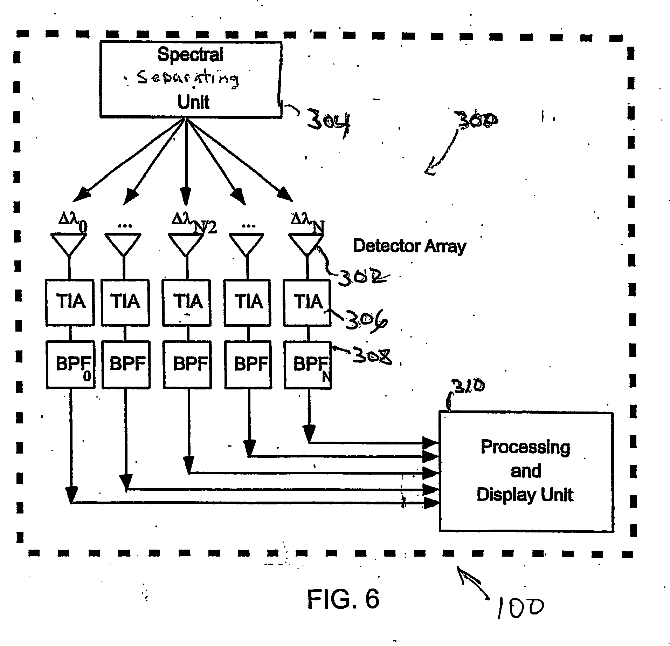

Processing of the multiple signals may be performed using an imaging or diagnostic console which performs basic operations including, mathematical image reconstruction, display, data storage. Alternatively, another embodiment, shown in Fig. 6, shows a standalone detection and processing system 300 that may be connected to OCT and/or LCI systems already in use. In this case, the detector 302 and digitization may be performed in the standalone unit. The input to the standalone unit would be the light combined from both reference and sample arms, as previously described. The output of the system would be an interferometric signal similar to previous OCT or LCI console inputs, but with increased SNR. The standalone unit would contain a splitter 304 for splitting the wavelengths into spectral bands, multiple detectors 302, analog electronics, including TIA's 306 and an arrangement for reconstructing the interferometric signal, as previously described. The arrangement for reconstructing the interferometric signal would include either analog or digital arrangement where the analog arrangement includes band pass filters ("BPF's") 308, and analog arrangement for adding the individual interferograms from each wavelength band. The digital arrangement would include an analog to digital converter, and a CPU 310 capable of recombining the interferograms from each spectral band into a single full bandwidth interferometric signal. The reconstructed interferogram may be then the output of the standalone system or alternatively, the reconstructed interferograms demodulated signal may be used as the input to the pre-existing system console.

Scan Range of the Reference arm.

The ranging depth in the sample 130 is determined by the resolution with which the cross spectral density can be determined. In a method using a single detector the spectral resolution of the complex spectral density is determined by the scan range of the reference arm. The larger the scan range, the higher the spectral resolution and the larger the ranging depth in the sample. In a system with a spectral separating unit and multiple detectors, the resolution of the cross spectral density is a combination of reference arm scan range and spectral separating characteristics.

Any suitable wavelength band shape may be used for separating. For arbitrary spectral band shapes, the scan range of the reference arm 18 is determined by the delay that is needed to completely resolve the spectral components in each band.

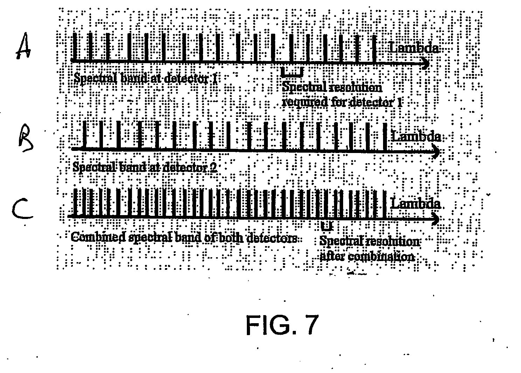

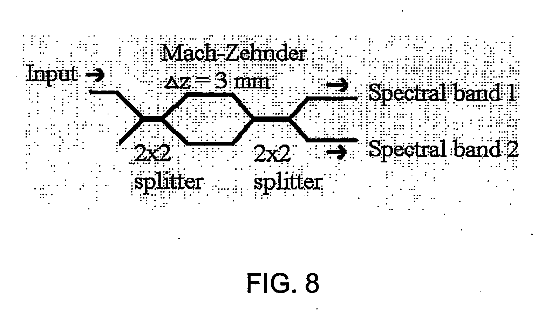

For instance, in one preferred embodiment, as depicted in Fig. 7, a spectral separating unit can split the spectrum into two bands where each band consists of a set of narrow spectra in a comb-like structure. Fig. 7A shows the spectral band at detector #1. Fig. 7B shows the spectral band at detector #2. Fig. 7C shows the combined spectral band of both detectors. Interleaving the comb-like spectral bands of each detector 24 gives back a continuous spectrum. The resolution needed to resolve the spectrum at an individual detector is half of what it would need to be in a single detector system, and thus the scan range of the reference arm can be reduced by a factor of two, while maintaining the same ranging depth in the sample 130. In an alternative embodiment, the spectral separating unit can be in the reference arm. In Fig. 8 an example is shown for splitting up the spectrum in several spectral bands. In this example the scan range of the reference arm can be reduced by a factor relating to the number of spectral bands while maintaining the same ranging depth in the sample.

Embodiments of the wavelength separating filter

Several techniques are known to separate or disperse the spectrum. One method uses a grating and a micro lens array to focus spectral components onto individual detectors. A second method uses prisms instead of a grating. A third method uses a grating and an addressable mirror array (such as, but not limited to, a "MEMS" mirror or digital light processing ("DLP") apparatus or the like) to direct spectral components to individual detectors. A fourth method uses a linear array of optical filters prior to the array of individual detectors. A fifth method uses waveguides etched into a material or manufactured from fiber optic components to generate a pattern with the desired filter action. As an example, in Fig. 8 an exemplary embodiment of a wave guide filter is provided that splits the spectrum into bands. A sixth method would use arrayed waveguide gratings ("AWG") to create the interleaved or arbitrary spectral bands.

Relative intensity noise

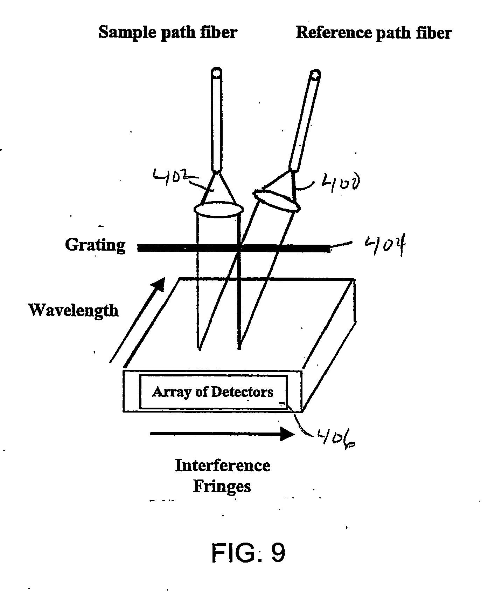

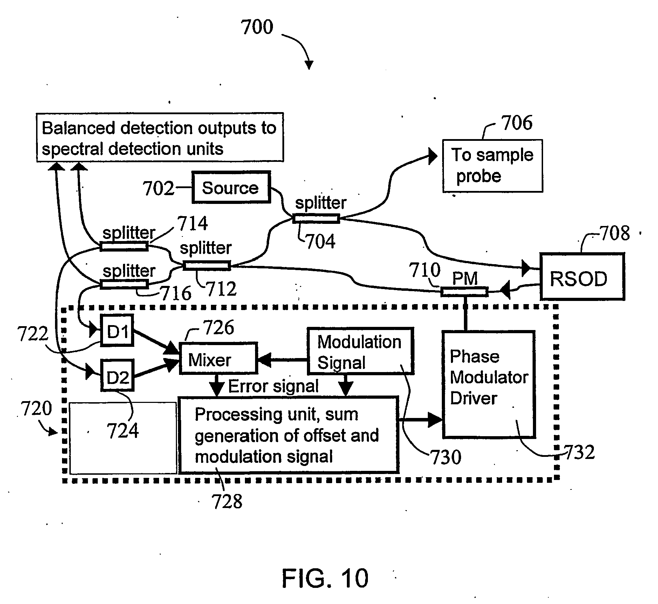

One of the noise terms that is present at the detectors is relative intensity noise ("RIN") or Bose-Einstein noise. RIN noise likely becomes dominant over shot noise for spectral widths less than a few nanometers. For many detector configurations, the spectral width at each detector may likely be smaller than a few nanometers, and the relative intensity noise can dominate the overall system noise. Thus, balanced detection, can preferably be implemented to eliminate the RIN. Several methods known in the art exist to implement balanced detection. One such method will be discussed below in further detail. For example, but not by way of limitation, as shown in Fig. 9, light from the reference arm 400 and sample arm 402 is incident on a grating 404 at slightly different angles and reflected and focused onto a linear N x M photo detector array 406. Along the N direction (column) of the array, wavelength is encoded. Along the M direction (row) of the array, the interference pattern of the sample and reference arm at a particular wavelength is recorded. Since sample and reference arm light were incident at slightly different angles, a pattern of interference maxima and minima will be present in the column direction. Balanced detection can be implemented by subtracting diode signals that are exactly out of phase with respect to the maxima and minima pattern. Alternatively, balanced detection can be implemented by measuring the amplitude of the interference pattern in the column direction which may be accomplished by subtracting the maxima or the interference pattern from the minima of the interference pattern along the column. An alternative embodiment for balanced detection is combining the reference and sample arm light 400, 402 to produce two outputs that have interference signals with a $\pi$ phase shift between them. This may be accomplished by taking both output ports of a beam splitter or other beam-recombining element. The two signals may then be detected separately and subtracted. Since the signals that contain the interference terms are shifted by $\pi$ in phase, these terms add constructively upon the operation of subtraction. The portion of signal that contains RIN, however, cancels upon subtraction. The subtraction operation can occur for all M elements and be conducted in the analog or digital domain. If subtraction is performed in the analog domain, the bandwidth of the signal is reduced by a factor of 2, preferably decreasing specified parameters of the digitization and data transfer across the computer bus.
An example of such balanced detection is shown in Fig 10, which is described more fully hereinbelow. The balance detection outputs are subtracted to generate a balanced signal that cancels RIN.

Signal processing to reconstruct the signal after spectral separating and detection.

Two cases will be discussed below as nonlimiting illustrations of exemplary embodiments of the present invention, firstly the case of continuous spectral bands (blocks), and secondly the comb-like spectral bands as depicted in Fig. 7

Case A: Continuous spectral bands.

The detection arm light is split into N spectral blocks, where each spectral block contains the intensity between two optical frequencies,

$$B_N = \int_{\omega_N}^{\omega_{N+1}} S_{ref}(\omega c / 2v)\, d\omega$$

(20)

The signal for the full spectral width is obtained by an FFT of the signal in each band, an optional compensation of dispersion and other corrections to the phase and amplitude of each Fourier component to optimize the signal and to correct the spectral density for side lobe reduction, addition of the complex FFT spectra, and inverse FFT on the added complex FFT spectrum, optionally with data reduction before the inverse FFT, to obtain the optionally demodulated function R(t), which is the interferometric response for a depth scan with the full source spectrum.

Case B1: Comb like spectral bands and the reconstruction of the full depth range in the sample arm from reduced reference arm scans.

The following description provided below describes the principle of reconstruction of the full depth range in the sample arm from reduced reference arm scans according to the present invention. The procedure shall be explained in the case of separating the spectrum in two spectral bands. The exemplary method can be expanded for separating into many spectral bands.

The signal at the detector for a single detector system is defined by R(t). The depth range in the sample is given by the measurement time T of a single A-line (depth profile) times the group velocity generated by the reference arm delay line, $Z_{range} = v_g T$

The smallest resolvable frequency after an FFT is given by 1/T, which gives a smallest resolvable angular frequency $\Delta\omega = 2\pi/T$. The filter as depicted in Fig. 8 splits the signal into two bands with peaks at $\omega=\omega_0, \omega_0 + 2\Delta\omega, \omega_0 + 4\Delta\omega$, *etc.* and $\omega= \omega_0 + \Delta\omega, \omega_0 + 3\Delta\omega$, *etc.*, respectively.

$B_1(t)$ and $B_2(t)$ are the signals in band one and two respectively. The signal in spectral bands one and two after Fourier transform are given by $B_1(\omega) = R(\omega)\cos^2(\omega T/4)$ and $B_2(\omega) = R(\omega)\sin^2(\omega T/4)$.

This product in the Fourier domain can also be written as a convolution in the time domain. Assuming the signals periodic with time T, the signals $B_1(t)$ and $B_2(t)$ are given by $B_1(t) = R(t) + R(t+T/2)$ and $B_2(t) = R(t) - R(t+T/2)$.

Using the above equations, the signal R(t) from t =0 to t=T can be reconstructed from the signals $B_1(t)$ and $B_2(t)$ recorded from t = 0 to t=T/2 by writing,

$R(t) = B_1(t) + B_2(t)$ and $R(t+T/2)= B_1(t)-B_2(t)$ for 0<t<T/2. For higher N>2, the identical procedure is performed such that R(t) is reconstructed from $B_1$ to $B_N$.

This demonstrates that the signals $B_1(t)$ and $B_2(t)$ only need to be recorded over half the depth range $Z_{range}$. Thus, the depth ranging in the reference arm can be reduced by a factor of 2, while the ranging depth in the sample remains the same. If the signal is split into more spectral bands, like shown in Fig. 7, a similar procedure as described above allows reduction of the depth scan in the reference arm by a factor of N, while the ranging depth in the sample remains the same, and N the number of spectral bands.

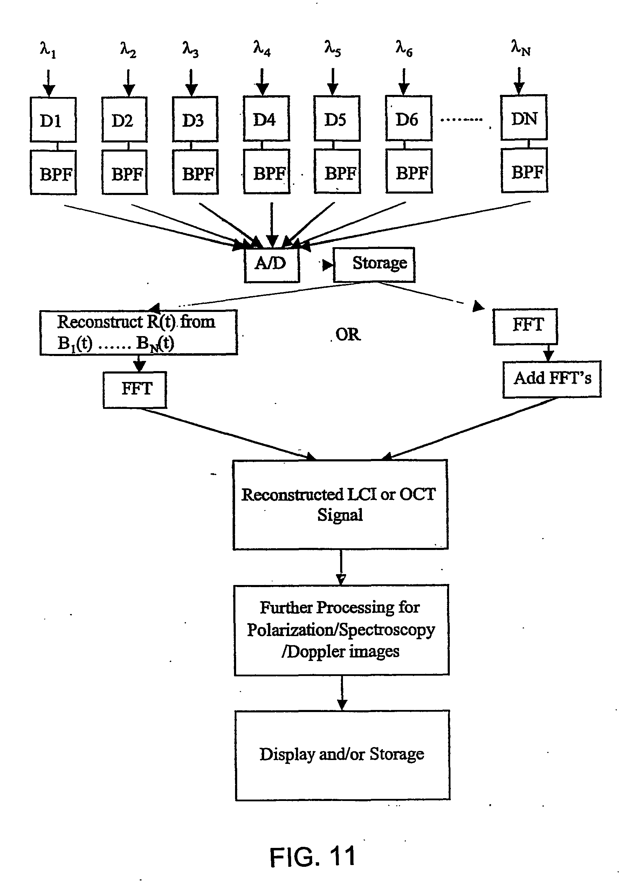

An exemplary flow diagram of the procedure described above is shown in Fig. 11.

Case B2. Limit of large number of spectral bands

In the limit of a large number of spectral bands, $N \geq \dfrac{L}{\lambda}$, the optical path length change in the reference arm approaches that of a wavelength, $\lambda$. In this limit, only a phase change across one wavelength is needed for reconstructing the entire axial scan over length L. In this case, the reference arm path delay may be accomplished by using any of the aforementioned ways for scanning the reference arm delay. Other preferred methods according to the present invention include insertion of an electrooptic modulator, acoustooptic modulator or phase control rapidly scanning optical delay line ("RSOD") in the reference arm path to impart the path length delay of one wavelength. Also in this case, the wavelength separating unit does not separate the wavelengths into a comb pattern, but separates the spectrum into unique optical frequencies, with each frequency detected by a single detector.

Case C. Fourier domain reconstruction for arbitrary wavelength patterns

In contrast to the reconstruction of the LCI or OCT signal in the time or space domains, the signal may be reconstructed in the Fourier domain by adding the complex spectral components for each wavelength band to compose the Fourier transform of the LCI or OCT signal. Alterations of the phase for each Fourier component may be preferred in certain selected circumstances to correct for minimization of reference arm delay length.

Reconstruction of the image or one dimensional axial scan

Following reconstruction of the LCI or OCT signal in the real domain, the axial reflectivity may be determined by demodulating the reconstructed LCI or OCT signal. An arrangement for demodulation can include multiplication by a sinusoid and low pass filtering, envelope demodulation using envelope detection, square law demodulation and low pass filtering, quadrature demodulation followed by FIR, IIR filtering, or low pass filtering. In addition, the reconstruction of Stokes vectors (polarization) and flow from these LCI or OCT signals is known to those skilled in the art. Following reconstruction and demodulation, the data may be displayed in one or two-dimensional format (image) for interpretation and ultimately diagnosis of a tissue condition or defect in a medium. If the LCI or OCT signal is reconstructed in the Fourier domain, such reconstructed signal in the Fourier domain can be demodulated in the Fourier domain by shifting the Fourier spectrum and performing an inverse Fourier transform. As a result, the complex signal in the real domain (quadrature signal) is then reconstructed into axial reflectivity information by computing the amplitude of the real portion of the quadrature signal. The complex component is used for computing polarization or flow information. Alternatively, if the signal is reconstructed in the Fourier domain, it can be directly inverse Fourier transformed into the real domain and undergo the aforementioned processing described for the reconstructed real domain signals.

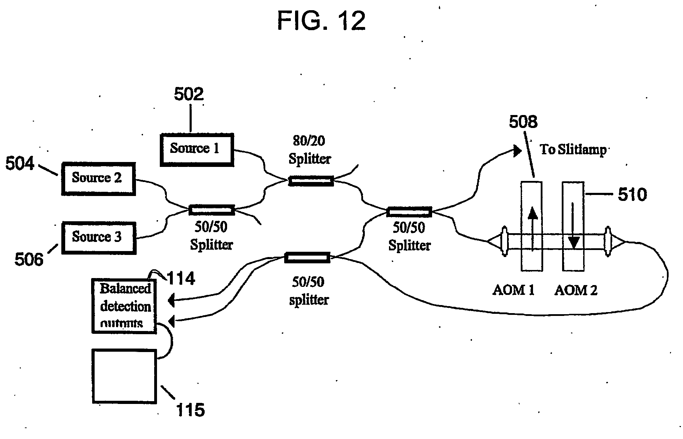

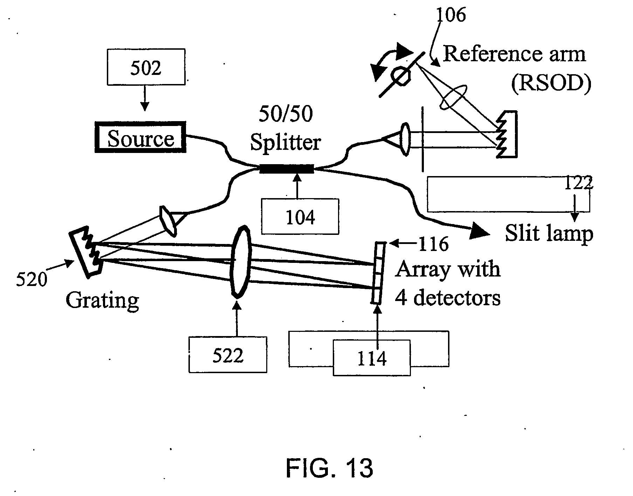

Fig. 12 shows an exemplary embodiment of a Spectral Domain OCT interferometer design 500 showing spectral compounding of light sources 502, 504, and 506 and acousto-optic generation of the carrier in the reference arm. The blocks labeled AOM are acousto-optic modulators 508, 510. The two outputs each go to separate spectral detection units 114, 115 (as depicted in Figs. 3 and 13) for balanced detection.

After spectral compounding of the source light in the first 50/50 splitter and the 80/20 splitter, light enters a modified Michelson interferometer. A configuration that implements balanced detection is shown. The sample arm goes to the probe (e.g., a slit lamp). Reference arm light is transmitted through two acousto-optic modulators with a difference frequency of 10 kHz to generate a constant carrier frequency that is independent of wavelength. The balanced detection outputs go to separate spectral detection units.

Spectral detection unit

Referring to Fig. 13, the core of Spectral Domain OCT is spectral separation of the detection arm light onto a multi-element array 114. The detection arm beam 520 is spectrally separated by a grating 520 and focused by a lens 522 onto a multi-element array 114.

A scan cameras with N detector elements is used as spectral detection unit 128 (see Fig. 2). Preferably, balanced detection is implemented by adding a second line scan camera. As is known to those skilled in the art, the depth range is inversely proportional to the spectral resolution. When the real part of the complex spectral density is determined, ranging depth z is defined by,

$$z = \frac{\lambda_0^2}{4\Delta\lambda}, \qquad\qquad (18)$$

Line scan rates of 20 kHz can be achieved, allowing demodulation of a 10 kHz carrier to extract the complex cross-spectral density. Data is digitized and transferred to computer memory. Demodulation of the signal is done in software. Scan rates of 10,000 depth profiles per second or more can be achieved.

Dual Balanced detection

Dual balanced detection is preferably used by the present invention, which is preferably utilized for the following reasons. Firstly, most light sources generate 1/f noise (f = frequency) at relatively low frequencies. Balanced detection will eliminate 1/f source noise. Secondly, an interference term of the sample arm light with itself (auto-correlation term) is present on top of the true signal term, which is the interference between sample and reference arm. This auto-correlation term can be eliminated by a differential technique. Balanced detection may eliminate this auto-correlation term from the measured signal. Thirdly, RIN can be reduced.

Data acquisition and processing unit

The data rate at 20,000 spectral profiles per second, with 2000 detector elements and 8-10 bit resolution (the dynamic range of most line scan cameras) is 40-80 MB/sec. Maximum sustainable data transfer speed over the PCI bus is 100 MB/sec. In a computer with two independent PCI bridges to computer system memory, approximately 200MB/sec of data can be transferred for real time processing of data from two line scan cameras simultaneously. Implementation of dual balanced detection in analog by subtracting line scan camera signals before digitization may reduce the data rate by a factor of 2. High-speed data acquisition boards are available at resolutions of 12-14 bits and speeds up to 100 Msamples/sec. A single 2048 point fast Fourier transform on a 2.5 GHz Pentium 4 processor takes 50 μsec. These numbers show that real-time processing of Spectral Domain OCT data at 20,000 spectral profiles/sec is within reach of current data acquisition and processing power of dual processor PC's. The data collected by the spectrometer can be sampled with equal wavelength increments. Fourier transform, however, links z and k space (or t and w). Because of the non-linear relation between k and λ the spectrum from the spectrometer should be interpolated to create evenly spaced samples in k domain. To achieve the optimal point spread function, dispersion in the sample and reference arm of the interferometer should be balanced. We have shown that dispersion imbalance can be corrected for by digital processing, allowing for correct compensation of dispersion for individual eye lengths.

**PHASE TRACING**

Apparatus and methods for phase tracking in spectral domain ("SD OCT are described.

**Fully parallel SD OCT**

One of the features of fully parallel SD OCT is spectral dispersion of the detection arm light onto a multi-element array such as but not limiting to an integrating device (e.g., CCD) and measurement of the real or complex spectral density at high speeds. The detection arm beam is separated by a spectral separating unit (e.g., grating) and focused onto the array. With respect to previous Spectral Domain OCT designs known in the art, two differences are apparent that will be discussed below: 1) implementation of balanced detection, and, 2) implementation of phase tracking.

**Spectrometer design** The depth range in SD OCT is inversely proportional to the spectral resolution. Using the complex spectral density, ranging depth z is given by,

$$z = \frac{\lambda_0{}^2}{2n\Delta\lambda}.$$

$$(18)$$

**Dual balanced detection**: Dual balanced detection is advantageous for at least three reasons. First, most light sources generate 1/f noise at relatively low frequencies (tens of kHz range). In time domain ("TD") OCT systems 1/f noise is not a problem because the signal carrier is in general in the MHz range where 1/f noise is not significant. In SD OCT, balanced detection may likely eliminate 1/f source noise. Second, an interference of the sample arm light with itself (auto-correlation term) is present on top of the true signal. This auto-correlation term can be eliminated by a differential technique. Balanced detection can be used to eliminate this auto-correlation term from the measured signal. Third, balanced detection may reduce relative intensity or Bose Einstein noise.

**Phase Tracking:** Phase tracking is preferable to eliminate phase instabilities in the interferometer. Phase instabilities can cause individual interferometric fringes to shift in location. If detection is slow relative to the shifting of the fringes, the resulting averaging results in an artifactual decrease in the measured fringe amplitude. Fast detection arrays can capture the cross spectral density at a rate of 20 to 40 kHz, resulting in integration times of 50 to 25 μsec, respectively. Phase instabilities arising on a time frame shorter than the integration time of the array should be compensated.

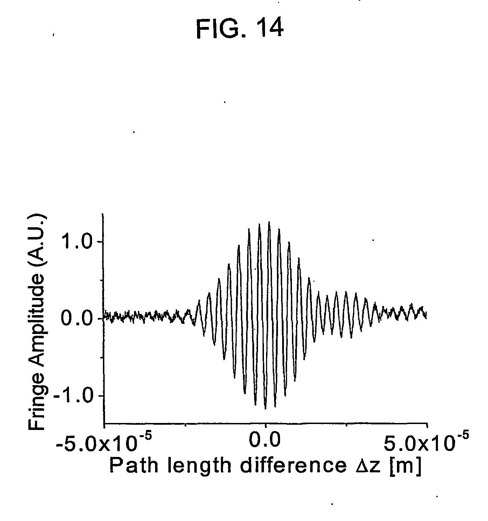

Fig. 14 shows an exemplary interference pattern as a function of path length difference between sample and reference arm.

Phase locking circuitry is common in electronics, and is frequently used in radar and ultrasound. Active phase tracking can be implemented by modulating the interferometer path length difference at 10 MHz with an electro-optic phase modulator in the reference arm over a fraction of the wavelength. By demodulating the intensity measured by one detector at the output of the interferometer at the frequency of the path length modulation, an error signal can be generated indicating in which direction the phase modulator should shift to lock onto a fringe amplitude maximum. By adding an offset to the phase modulator as determined by the error signal, the phase tracker actively locks onto a fringe maximum. The phase modulator can only modulate the path length difference over a few wavelengths. The processing unit can determine if the phase modulator has reached its range limit, and jump by a full wave in phase to maintain lock on a different fringe maximum. This approach exploits the fact that phase should be controlled only modulo 2π In addition, the processing drives a slower component (e.g., the Rapid Scanning Optical Delay line) to extend the path length range of the phase modulator/RSOD combination over several millimeters. Phase locking can be performed on a fringe maximum, minimum, or zero crossing, based on the type of mixing performed in the demodulation circuit.

Autoranging technology can also be used, including processing algorithms, as disclosed in copending U.S. application No. 10/136,813, filed April 30, 2002, entitled METHOD AND APPARATUS FOR IMPROVING IMAGE CLARITY AND SENSITIVITY IN OPTICAL COHERENCE TOMOGRAPHY USING DYNAMIC FEEDBACK TO CONTROL FOCAL PROPERTIES AND COHERENCE GATING.

The autoranging mechanism may comprise a processor unit for (a) obtaining a first scan line; (b) locating a surface location "S" of a sample; (c) locating an optimal scan range "R" of the sample;(d) modifying a reference arm delay waveform to provide an output; (e) outputting the output to a reference arm; (f) determining whether the image is complete; and (g) moving to the next scan line if the image is not complete or remapping the image using the surface S data and the waveform data stored in the memory storage device if the image is complete.

If the light returned from the sample is of low amplitude, phase locking may be unstable due to the presence of noise. In another embodiment, a separate, preferably monochromatic, light source is input into the interferometer. The separate source wavelength may overlap with the broad bandwidth OCT or LCI source spectrum or may be centered at a different wavelength than the OCT or LCI source spectrum. The separate source is preferably of higher power and may be combined with the source arm (using wavelength division multiplexer, grating, prism, filter or the like) travel to the reference and sample arms and return back to the beam recombining element. The returned separate source light can then separated from the OCT or LCI light following transmission back through the beam recombining element (i.e. beam splitter output). A separation arrangement can perform spectral separation by a dispersing element, such as a dichroic mirror, filter, grating, prism, wavelength division multiplexer or the like. The separate source will be detected separately from the OCT or LCI broad bandwidth light using one or more detectors. The higher power provided by this separate source can enable detection of a higher amplitude interference pattern, and provide an improved input to the phase tracker, thus enabling more stable phase tracking.

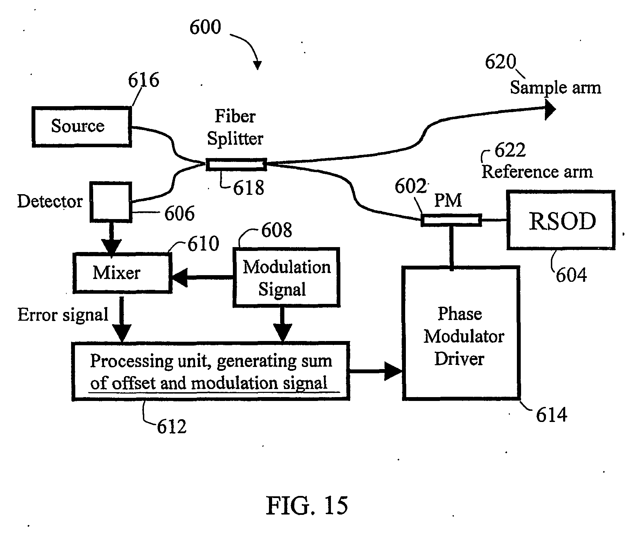

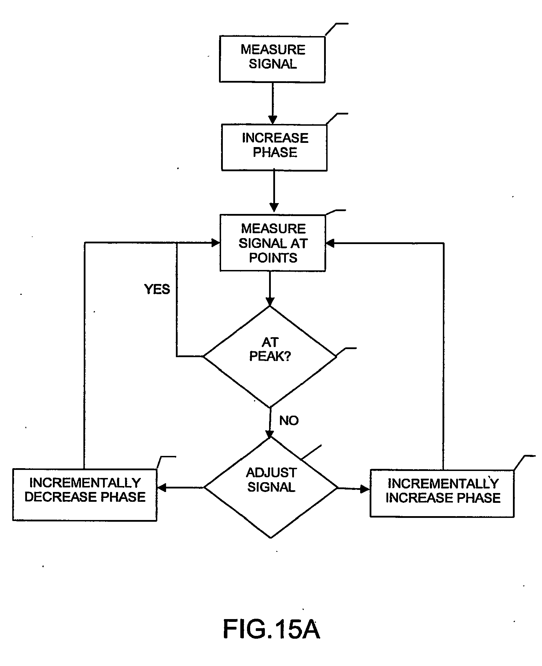

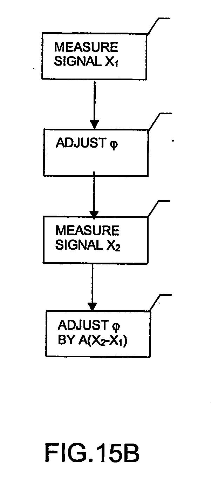

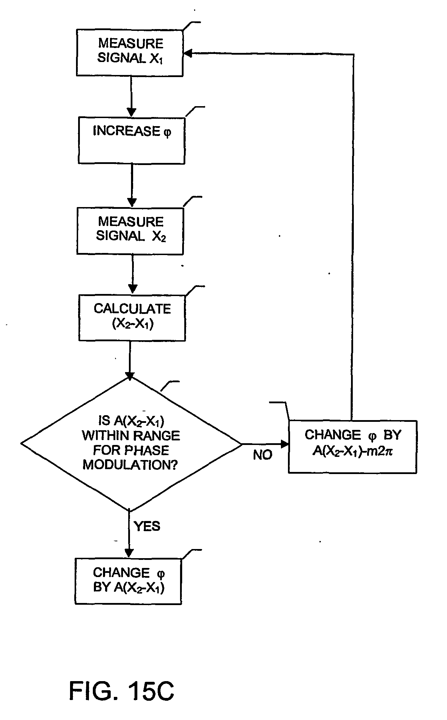

Fig. 15 shows one exemple of a phase tracker system 600 with an extended phase lock range, by combining a fast element (EO phase modulator) 602 to modulate the path length difference over a small range, and a slower element (RSOD) 604 to modulate the path length over an extended range. The detector 606 signal is mixed with the phase modulator modulation frequency 608 by a mixer 610 and low pass filtered (filter not shown) to generate an error signal. The processing unit 612 preferably processes the error signal to generate an offset voltage, and adds this offset voltage to the modulation signal 608, so as to generate the output for the phase modulator driver 614. In addition, the processing unit 612 can generate a signal to the RSOD 604 to provide extended range tracking of the phase over distances of several millimeters. Light source 616, fiber splitter 618, sample arm 620 and reference arm 622 are shown, and are described herein.

**Mixer Implementation:** The intensity I(t) at the detector at a given moment within a single oscillation of the fringe pattern is given by

$$I(t) = \cos[\varphi(t)]$$

where the phase φ gives the position in the fringe. For φ=0, the signal is at a fringe maximum, for φ=π, the signal is at a fringe minimum. At an arbitrary moment t, the phase φ(t) is given by,

$$\varphi(t) = \alpha + \beta \sin(\omega t)$$

where α describes the position within a single oscillation of the fringe pattern, and β*sin(ωt) is the phase modulation introduced by the phase modulator, with β the amplitude of the phase modulation, and ω the frequency of the phase modulation signal. The intensity at the photodetector I(t) can be mixed with a carrier at frequency ω and 2ω, resulting in the mixer signal MixerC(t), MixerS(t), Mixer2ωC(t) and Mixer2ωS(t),

$$MixerC(t) = \cos(\omega t) * \cos(\alpha + \beta \sin(\omega t)); \quad MixerS(t) = \sin(\omega t) * \cos(\alpha + \beta \sin(\omega t))$$

$$Mixer2\omega C(t) = \cos(2\omega t) * \cos(\alpha + \beta \sin(\omega t));$$

$$Mixer2\omega S(t) = \sin(2\omega t) * \cos(\alpha + \beta \sin(\omega t))$$

The time average over a single oscillation of the carrier frequency ω of MixerC, MixerS, Mixer2ωC and Mixer2ωS is given by,

$\overline{MixerC(t)} = 0$; $\overline{MixerS(t)} = \sin(\alpha)* J_1(\beta)$ $\overline{Mixer2\omega C(t)} = \cos(a) *J_2(\beta)$ ; $\overline{Mixer2\omega S(t)} = 0$

where $J_1(\beta)$ and $J_2(\beta)$ are a Bessel functions of the first kind; its value depends on β, the amplitude of the phase modulation. Thus, the signal $\overline{MixerS(t)}$ and $\overline{Mixer2\omega C(t)}$ are proportional to sin(α) and cos(α), respectively, with α the position within a single oscillation of the fringe pattern. The mixer outputs $\overline{MixerS(t)}$ and $\overline{Mixer2\omega C(t)}$ are used as an error signal to generate an offset voltage to steer the phase modulator to a new center position that miniznizes the error signal, and locks the interferometer output on a fringe maximum or minimum, or a zero crossing, respectively. The complex spectral density can now be determined by two consecutive array scans, one where the error signal sin(α) is minimized, and the next where the error signal cos(α) is minimized, resulting in a 90 degrees phase shift between the two interference patterns. Using this mixing arrangement, the complex spectral density can be obtained rapidly and without resorting to an additional mechanical arrangement for changing the phase of the reference arm light

Fig. 10 shows one exemple of a SD OCT system 700 with phase tracker for providing balanced detection. In this example a source 702 provides light which passes through a splitter 704, which sends part of the light to a sample probe 706 and the remainder of the light to a Rapid Scanning Optical Delay ("RSOD") line 708. Light is passed from the RSOD 708 to the phase modulator PM 710. Light from the phase modulator PM 710 is sent through a splitter 712, and then through two additional splitters 714 and 716, a portion of the output of which is sent as balanced detection outputs to spectral detection units (not shown, but as described elsewhere herein) and the remainder of the output is sent to the phase tracker assembly 720. In the phase tracker assembly 720, phase tracker detectors $D_1$, and $D_2$, 722 and 724, receive the partial output of the pair of splitters 714 and 716, which in turn send signal to a mixer 726 to generate an error signal. A processing unit 728 processes the error signal, where the sum generation of offset voltage and adds this to the modulation signal 730 to generate the output for the phase modulator driver 732. Modulation signal, shown at box 730, is forwarded to the mixer 726 and the processing unit 726. In addition, the fringe amplitude could be too small for the phase tracker to lock. Alternatively, a secondary source with longer coherence length could be coupled to the system 700 to provide a larger fringe amplitude to the phase tracker.

A method for tracking phase in an imaging system is described as shown in Figs. 15A-C the method comprising the steps of: (a) measuring a signal received from the sample arm; (b) increasing a phase of the signal; (c) measuring a first signal partition of the signal defined as $x_1$ at at least one peak of the signal; (d) determining whether to increase or decrease the phase of the signal by an incremental amount; (e) after step (d), measuring a second signal partition of the signal following step d); and, if the signal is at its peak, remeasuring the signal and if the signal is not at its peak, repeating steps d) and e).

The method further may comprise that steps (a) - (f) are performed in parallel with other imaging processes. The adjustment of phase "φ" is defined as $A(x_2-x_1)$, where "A" is a constant. Furthermore, optionally, step d) may further comprise the substeps of d1) determining whether $A(x_2-x_1)$ is within range of the phase modulator, and d2) changing φ by an amount equal to $A(x_2-x_1)$ if $A(x_2-x_1)$ is within the range or changing φ by an amount equal to $A(x_2-x_1)-m2\pi$ if $A(x_2-x_1)$ is outside of the range, where M is an integer greater than 1. The method may optionally further comprise a substep d3) remeasuring signal $x_1$.

**Data acquisition and processing unit**

In general, the data collected by the spectrometer are sampled with equal wavelength increments. Fourier transform, however, links z and k space (or t and w). Because of the non-linear relation between k and λ the acquired spectrum is interpolated to create evenly spaced samples in the k domain. Alternatively, the light could be dispersed in such a way on the detection array that the light is samples in equal intervals in k space, such that the interpolation becomes obsolete. Alternatively, the detection array spacing could be designed to sample the light evenly spread in the k domain, such that the interpolation becomes obsolete. To achieve the optimal point spread function, dispersion in the sample and reference arm of the interferometer should preferably be balanced. Dispersion imbalance can be corrected by digital processing.

A probe for locating atherosclerotic plaque in a blood vessel is described comprising: an interferometer; a spectral separating unit which splits signal received from the interferometer into a plurality of optical frequencies; and a detector arrangement capable of detecting at least a portion of the optical frequencies received from the spectral separating unit.

An apparatus for delivering a therapeutic agent is described, comprising: a probe disposed in the housing and comprising: an interferometer, a spectral separating unit which splits signal received from the interferometer into a plurality of optical frequencies, a detector arrangement capable of detecting at least a portion of the optical frequencies received from the spectral separating unit; and a conduit cooperating with the probe, and comprising a proximal end for receiving the therapeutic agent and a distal end for delivering the therapeutic agent at a predetermined location, the location being determined by imaging the environment in proximity to the distal end using the probe.

An exemplary embodiment of the present invention will be further described below in connection with the following example, which is set forth for purposes of illustration only.

EXAMPLE

The method according to the present invention was verified in the laboratory by the following experiment.

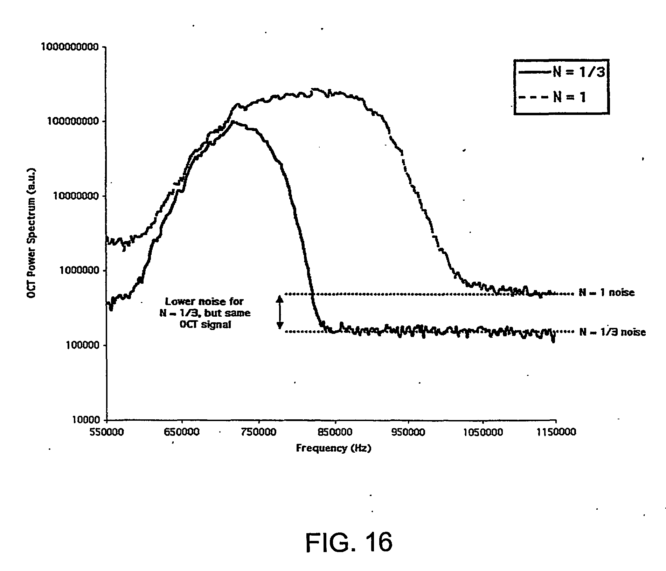

In the existing OCT system, the shot noise power spectrum as determined from the spectral density due to the reference arm optical power was measured. Then 2/3 of the spectrum from the reference arm was blocked, and experimentally it was verified that the shot noise power spectrum was reduced by a factor of three, thus demonstrating that the shot noise is reduced by a factor of 3 if the spectrum is split in three spectral bands (see Fig. 16). The upper curve (gray dotted line) shows the power spectrum for the OCT signal with one detector. For the lower curve (solid line), the spectrum was limited by 1/3 with a corresponding factor of 3 improvement in signal to noise ratio. This data was generated by experiment, blocking 2/3 of the spectrum in a grating-based double-passed pulse shaping rapidly scanning optical delay line.

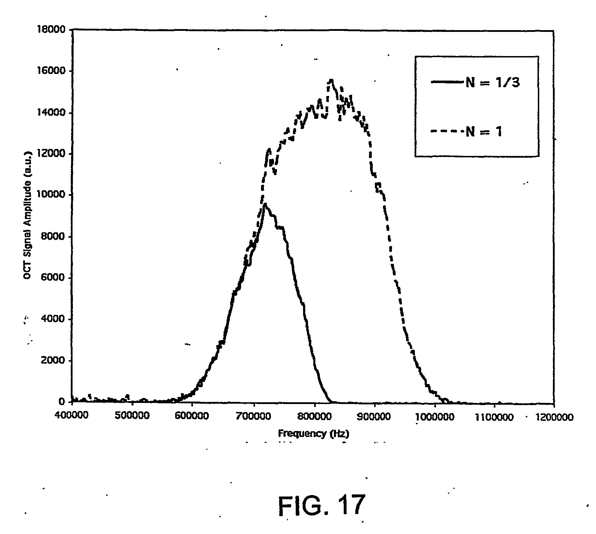

An object with low reflectivity was inserted in the sample arm. Using the full spectral width of the source, the power spectrum of the interference between sample and reference arm light was determined in the lower half of the spectral density. Then the upper part of the source spectrum was blocked in the reference arm, and it was verified that the lower 1/3 of the power spectrum of the interference between sample and reference arm light had the same magnitude as in the previous measurement (see Fig. 17). This figure demonstrates that the signal amplitude is equal for the N=1 and N=1/3 cases where they overlap. The result of equal amplitude signal for N=1/3 case and the 3-fold lower noise for the N=1/3 case (see Fig. 6) demonstrates that splitting into N wavelength bands increases the SNR by a factor of N.

This demonstrates that when the light in the detection arm is split in two spectral bands, the spectral density of the interference between sample and reference arm light within the spectral bandwidth of a single detector is unchanged. Combined with the measurement that showed a reduction in the shot noise power spectrum, the conclusion is that a reduction of shot noise can be realized by splitting the detection arm light in separate spectral bands.

Experimental verification of the noise reduction.

To demonstrate the noise reduction in Spectral Domain OCT, an OCT system was used, including a Rapid Scanning Optical Delay line (RSOD) was used in the reference arm, enabling portions of the spectrum to be blocked. Detector signals were digitized at 2.5 Msamples/sec, allowing digital processing of the fringe information. First, the thermal noise density of the detector was measured as a function of frequency by blocking all light onto the detector. Second, the shot noise density of the reference arm power was measured with only the reference arm power incident on the detector. Third, both the sample and reference arm light were incident on the detector. The sample was a single scattering surface mounted in a model eye and 512 depth profiles were acquired in 2. seconds. The power density $I(f)^2$ was measured, which is proportional to the spectral density squared (see Eq. (9)). Then we blocked half of the spectrum in the reference and measured again the shot noise density of the reference arm by blocking the sample arm, and the power density $I(f)^2$ when both sample and reference arm light were incident on the detector. Shot noise and power densities were corrected for thermal noise by subtraction. Thermal noise was at least a factor of 3 smaller than the lowest shot noise level.

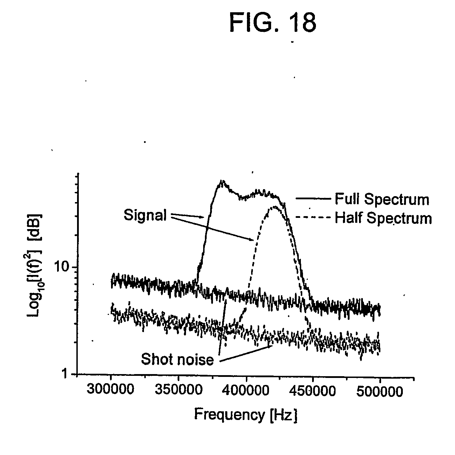

Fig. 18 shows a graph of power density for the full spectrum, and with half of the spectrum blocked in the reference arm, as a function of frequency. The solid line shows the power density for the full spectrum. The shot noise level measured while the sample arm was blocked is also shown. The dashed line shows the power density with half the spectrum blocked in the sample arm. The shot noise level measured while the sample arm was blocked is also shown. Fig. 18 demonstrates that the shot noise level was reduced by a factor of 2 by blocking half the spectrum in the reference arm. At the same time, the signal at frequencies corresponding to wavelengths that were not blocked in the reference arm remained the same.

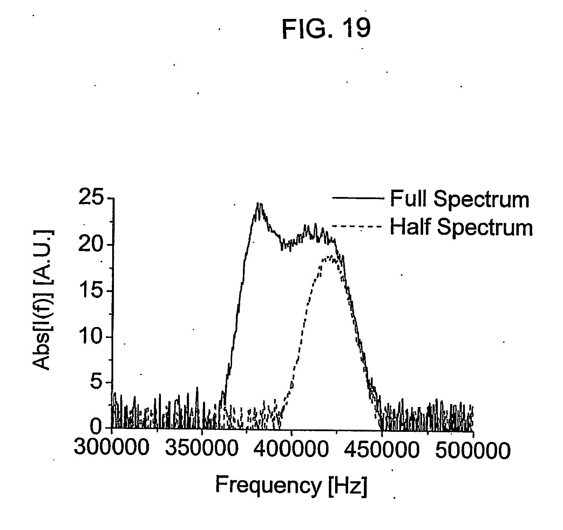

As is evident from Fig. 18, which summarizes the measured results, the shot noise density is reduced by approximately a factor of 2 by blocking half the spectrum in the reference arm. Fig. 19 shows that after subtraction of the shot noise levels from the corresponding signals, the power densities for those frequencies that corresponded to wavelengths that were not blocked in the reference arm remained the same. This demonstrates that the shot noise density is reduced by a factor of 2 when the total reference arm power is reduced by a factor of 2 by blocking half the spectrum, while the signal power density for wave lengths not blocked in the reference arm remains unchanged.

Fig. 19 shows a graph of the square root of the power densities for the full spectrum, and for half the spectrum blocked in the reference arm as a function of frequency. The solid line shows the spectrum after subtraction of the respective shot noise. The dashed line shows the half spectrum after subtraction of the respective shot noise. Fig. 13 demonstrates that after subtracting the respective shot noise contributions, the signal at frequencies corresponding to wave lengths that were not blocked in the reference arm remained the same.

The next experiment further demonstrated that by dispersing the spectrum in the detection arm over several detectors, and by selectively band pass filtering the signals of each detector, the SNR is increased. The detection arm light was dispersed over 4 detectors by a diffraction grating as shown in Fig. 13, and the detector signals were separately amplified by transimpedance amplifiers with a bandwidth of 600 kHz and simultaneously digitized.

Fig. 13 shows a schematic of an exemplary apparatus setup used to demonstrate SNR improvement by Spectral Domain OCT according to the present invention. Scanning of the reference arm 106 was performed with a Rapid Scanning Optical Delay line (RSOD) 120. Individual signals from the array detector 114 were amplified by transimpedence amplifiers, digitized by a 4-channel 2.5 MHz per channel A/D board and stored in computer memory (not shown).

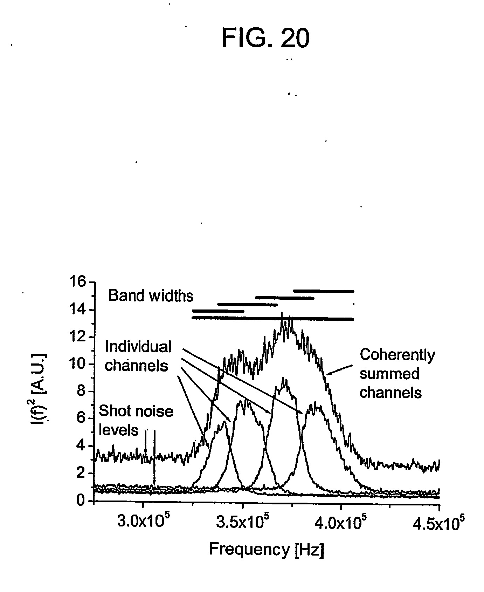

First, the thermal noise density of all four detectors was measured. Second, the shot noise density of the reference arm light in each detector channel 116 was measured. Third, both the sample and reference arm light were incident on the detector 114. The sample 130 was a single scattering surface mounted in a model eye and 512 depth profiles were acquired in 2 seconds. The power density $I(f)^2$ in each detector channel 114 was measured. Then, the signals of the four detectors 114 were summed, and the combined power density $I(f)^2$ was determined. The results are shown in Fig. 20, which demonstrates that the shot noise is lower in each individual channel compared with the sum of all channels, but that the power densities $I(f)^2$ in the individual channels within their respective bandwidths are approximately equal to the power density $I(f)^2$ of the coherent sum of the four channels.

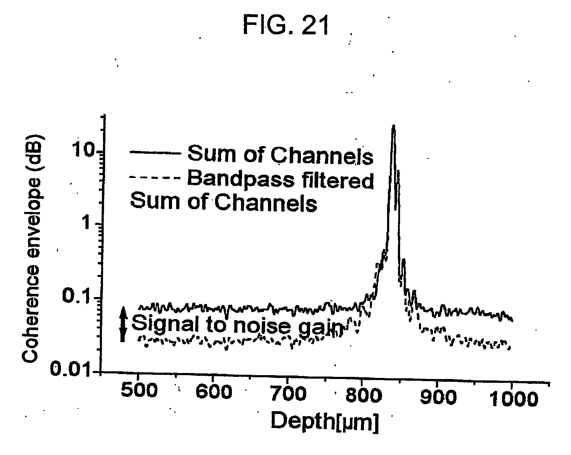

Fig. 20 shows a graph of the power densities for four separate detectors 116 of Fig. 13. The spectrum in the detection arm was dispersed over four separate detectors 116 by a diffraction grating 520. The shot noise levels for each individual detector 116 are significantly lower than for the coherent sum of the four detector channels. Bars at the top of the image indicate the signal pass band that was applied to the individual channels and the coherently summed channel to generate Fig. 21.

In Fig. 21, the square of the coherence envelope is shown for both the direct sum of all four detection channels and the coherent sum after digitally band pass filtering each detector channel with a bandwidth centered at the center frequency of the respective detector signal. Fig. 21 shows that the interference fringe signal $I(t)$ of the direct sum and the band pass filtered coherent sum of the four detector signals results in virtually the same coherence envelope peak value, while the band pass filtered coherent sum of the four detector signals shows a significantly lower noise level than the direct coherent sum. Since the pass band of each individual channel was slightly larger than one third of the pass band of the full signal (pass bands are indicated in Fig. 20), an increase of SNR of a factor of 2.87 was expected. The noise level dropped by a factor of 2.8. However, band pass filtering also reduced the signals slightly, by a factor of 1.12, resulting in an effective increase in SNR of a factor of 2.5.

These experiments clearly demonstrate that spectrally dispersing the light in the detection arm can offer a significant SNR advantage.

Fig. 21 shows a plot of the coherence envelope for the coherently summed channels, and the coherently summed channels after band pass filtering each channel. The solid line is the sum of channels. The dashed line is the pass filtered sum of channels. Fig. 21 clearly demonstrates the signal to noise gain that can be achieved by spectrally dispersing the signal in the detection arm over several individual detectors. In this example the noise level was reduced by a factor of approximately 2.8. Since the coherence peak was reduced by a factor of 1.12 due to some remaining signal fraction filtered out by the band pass filters, the actual SNR improvement was 2.5.

**Claims**

1. An apparatus (100) for optical imaging, comprising:

   a) a low coherence light source;
   b) an interferometer (102) comprising a reference arm , a sample arm, and a means for recombining reference arm light and sample arm light;
   c) a motionless non mechanical arrangement for introducing a reference arm delay;
   d) a spectral separating unit (112) which splits a signal received from said interferometer into a plurality of spectral bands;
   e) a plurality of detectors (114), each detector being arranged to detect at least a portion of the spectral bands received from the spectral separating unit, the detectors being configured to generate signals as a function of the detected spectral bands; and
   f) a signal processing unit (122) which is configured to process the signals so as to determine a depth profile of the sample as a function of the signals.

**2.** The apparatus according to claim 1, wherein said motionless non-mechanical arrangement comprises a Rapid Scanning Optical Delay line.

**3.** The apparatus according to claim 1, wherein each of the detectors is configured to detect a cross spectral density of a respective one of the spectral bands, and wherein the signal processing unit is further configured to combine the cross spectral density detected by each of the detectors so as to determine a full cross spectral density.

**4.** The apparatus according to claim 3, wherein the signal processing unit is configured to determine the depth profile as a function of the determined full cross spectral density.

**5.** The apparatus according to claim 1, wherein the reference arm light is received from a transmissive reference.

**6.** The apparatus according to any one of the preceding claims, further comprising a splitter configured to combine the reference arm light and the sample arm light.

**7.** The apparatus according to any one of the preceding claims, wherein the detectors comprise one of (i) at least one single-dimensional detector array, and (ii) at least one multi-dimensional detector array.

**8.** The apparatus according to any one of the preceding claims, wherein the spectral separating unit comprises at least one of (i) at least one reflection grating, (ii) at least one transmission grating, and (iii) at least one spectrally dispersive component.

**9.** The apparatus according to any one of the preceding claims, further comprising at least one charge coupled device coupled to the plurality of detectors.

**10.** The apparatus according to any one of the preceding claims, further comprising at least one bandpass filter coupled to the plurality of detectors.

**11.** The apparatus according to claim 10, wherein the bandpass filter is an electronic bandpass filter.

**12.** The apparatus according to any one of the preceding claims, further comprising at least one analog to digital converter coupled to the plurality of detectors.

**13.** The apparatus according to claim 1, wherein the detectors are provided as a single detection array.

**14.** A method for optical imaging using an apparatus according to any preceding claim, comprising the steps of:

receiving a reference arm light from the reference arm of the interferometer and sample arm light from the sample arm of the interferometer, introducing a delay in the reference arm light, and combining the delayed reference arm light and the sample arm light;
splitting the combined light into a plurality of spectral bands;
with each of the detectors, detecting at least a portion of the spectral bands;
generating signals as a function of the detected spectral bands; and
processing the signals, and determining a depth profile of the sample as a function of the signals.

**15.** The method according to claim 14, further comprising:

detecting a cross spectral density of a respective one of the spectral bands; and
combining the cross spectral density detected by each of the detectors so as to determine a full cross spectral density.

**16.** The method according to claim 15, comprising;
determining the depth profile as a function of the determined full cross spectral density.

**Patentansprüche**

**1.** Vorrichtung (100) zur optischem Abbindung mit:

a) einer mit geringer Kohärenz,

b) einem Inteferometer (102) mit einem Referenzarm, einem Probenarm und einem Mittel zum Rekombinieren des Referenzarmlichts und des Probenarmlichts,

c) eine bewegungslose nicht-mechanische Unordnung zum Einfügen einer Referenzarmverzögerung,

d) einer Spektralteilereinheit (112), die ein von dem Interferometer empfangenes Signal in eine Mehrzahl von Spektralbändern aufteilt,

e) eine Mehrzahl von Detektoren (114), wobei jeder Detektor so eingereichtet ist, dass er mindestens einen Teil der von der Spektralteilereinheit empfangenen Spektralbänder erfasst, wobei die Detektoren so eingerichtet sind, dass sie Signals als eine Funktion der erfassten Spektralbänder erzeugt, und

f) einer Signalverarbeitungseinheit (122), die so eingerichtet ist, dass sie die Signale so verarbeitet, dass ein Tiefenprofil der Probe als eine Funktion der Signale bestimmt wird.

2. Vorrichtung nach Anspruch 1, wobei die bewegungslose nicht-mechanische Anordnung eine schnell scannende optisch Verzögerungsstrecke aufweiset.

3. Vorrichtung nach Anspruch 1, wobei jeder der Detektoren so eingereichtet ist, dass er eine spektrale Querschnittsdichte eines entsprechenden der Spektralbänder erfasst, und wobei die Signalverarbeitungseinheit darüber hinaus so eingereichtet ist, dass sie die von jedem der Detektoren erfasste spektrale Querschnittsdichte kombiniert, sodass eine vollständige spektrale Querschnittsdichte bestimmt wird.

4. Vorrichtung nach Anspruch 3, wobei die Signalverarbeitungseinheit so eingerichtet ist, dass sie das Tiefenprofil als eine Funktion der bestimmten vollständigen spektralen Querschnittsdichte bestimmt.

5. Vorrichtung nach Anspruch 1, wobei das Referenzarmlicht von einer lichtdurchlässigen Referenz empfangen wird.

6. Vorrichtung nach einem der vorhergehenden Ansprüche, darüber hinaus mit einem Teiler, der so eingerichtet ist, dass er das Referenzarmlicht und das Probenarmlicht kombiniert.

7. Vorrichtung nach einem der vorhergehenden Ansprüche, wobei die Detektoren eines aufweisen aus (i) mindestens einem eindimensionalen Detektorarray und (ii) mindestens einem mehrdimensionalen Detektorarray.

8. Vorrichtung nach einem der vorhergehenden Ansprüche, wobei die Spektralteilereinheit mindestens eines aufweist aus (i) mindestens einem Reflexionsgitter, (ii) mindestens einem Transmissionsgitter und (iii) einer spektraldispersiven Komponente.

9. Vorrichtung nach einem der vorhergehenden Ansprüche, darüber hinaus mit mindestens einer ladungsträgergekoppelten Schaltung, die mit der Mehrzahl von Detektoren verbanden ist.

10. Vorrichtung nach einen der vorhergehenden Ansprüche, darüber hinaus mit mindestens einem Bandpassfilter, das mit der Mehrzahl von Detektoren verbunden ist.

11. Vorrichtung nach Anspruch 10, wobei das Bandpassfilter ein elektronisches Bandpassfilter ist.

12. Vorrichtung nach einen der vorhergehender Ansprüche, darüber hinaus mit mindestens einem Analog.Digital-Wandler, der mit der Mehrzahl von Detektoren verbunden ist.

13. Vorrichtung nach Anspruch 1, wobei die Detektoren als ein einziges Detektionsarray vorgegehen sind.

14. Verfahren zum optischen Abbilden, wobei eine Vorrichtung nach einem der vorhergehenden Anspruche verwendet wird, mit den Schritten:

Empfanden eines Referenzarmlichts aus dem Referenzarm des Interferometers und eines Probenarmlichts aus dem Probenarm des Interferometers, Einfügen Verzögerung des Referenzarmlichts und Kombinieren des verzögerten Referenzarmlichts und des Probenarmlichts,
Teilen des kombinierten Lichts in eine Mehrzahl von Spektralbändern,
wobei jeder der Detektoren mindestens einen Teil der Spektralbänder erfasst,
Erzeugen von Signalen als eine Funktion der erfassten Spektralbänder und
Verarbeiten der Signale und Bestimmen eines Tiefenprofils der Probe als eine Funktion der Signale.

**15.** Verfahren nach Anspruch 14, darüber hinaus mit:

Erfassen einer spektralen Querschnittsdichte eines entsprechenden der Spektralbänder und
Kombinierten der spektralen Querschnittsdichten, die von jedem der Detektoren erfasst werden, sodass vollständige Querschnittsdichte bestimmt wird.

**16.** Verfahren nach Anspruch 15 mit:

Bestimmen des Tiefenprofils als eine Funktion der bestimmten vollständigen spektralen Querschnittsdichte.


**Revendications**

**1.** Appareil (100) d'imagerie optique, comprenant :

a) une source lumineuse à faible cohérence ;
b) un interféromètre (102) comprenant un bras de référence, un bras d'échantillon, et un moyen pour recombiner une lumière de bras de référence et une lumière de bras d'échantillon ;
c) un agencement non mécanique dépourvu de mouvement pour introduire un retard de bras de référence, ;
d) une unité de séparation spectrale (112) qui divise un signal reçu à partir dudit interféromètre en une pluralité de bandes spectrales ;
e) une pluralité de détecteurs (114), chaque détecteur étant agencé pour détecter au moins une partie des bandes spectrales reçues à partir de l'unité de séparation spectrale, les détecteurs étant configurés pour générer des signaux en fonction des bandes spectrales détectées ;
f) une unité de traitement de signaux (122) qui est configurée pour traiter les signaux afin de déterminer un profil de profondeur de l'échantillon en fonction des signaux.

**2.** Appareil selon la revendication 1, dans lequel ledit agencement non mécanique dépourvu de mouvement comprend une ligne à retard optique à balayage rapide (« Rapid Scanning Optical Delay »).

**3.** appareil selon la revendication 1, dans lequel chacun des détecteurs est configuré pour détecter une densité spectrale croisée d'une bande respective des bandes spectrales, et dans lequel l'unité de traitement de signaux est en outre configurée pour combiner la densité spectrale croisée détectée par chacun des détecteurs afin de déterminer une densité spectrale croisée complète.

**4.** Appareil selon la revendication 3, dans lequel l'unité de traitement de signaux est configurée pour déterminer le profil de profondeur en fonction de la densité spectrale croisée complète déterminée.

**5.** Appareil selon la revendication 1, dans lequel la lumière de bras de référence est reçue à partir d'une référence transmissive.

**6.** Appareil selon une quelconque des revendications précédentes, comprenant en outre un diviseur configuré pour combiner la lumière de bras de référence et la lumière de bras d'échantillon.

**7.** selon une quelconque des revendications précédentes, dans lequel les détecteurs comprennent un élément parmi (i) au moins une matrice monodimensionnelle de détecteurs, et (ii) au moins une matrice multidimensionnelle de détecteurs.

**8.** Appareil selon une quelconque des revendications précédentes, dans lequel l'unité de séparation spectrale comprend au moins un élément parmi (i) au moins un réseau de réflexion, (ii) au moins un réseau de transmission, et (iii) au moins un composant dispersif de spectrale.

**9.** Appareil selon quelconque des revendication précédentes, comprenant en outre au moins un dispositif à couplage de charges couplé à la pluralité de détecteurs.

**10.** Appareil selon une quelconque des revendications précédentes, comprenant en outre au moins un filtre passe-bande couplé à la pluralité de détecteurs.

**11.** Appareil selon la revendication 10, dans lequel le filtre passe-bande est un filtre passe-bande électronique.

**12.** Appareil selon une quelconque des revendications précédentes, comprenant en outre au moins un convertisseur analogique-numérique couplé à la pluralité de détecteurs.

**13.** Appareil la revendication 1, dans lequel les détecteurs sont prévus sous forme de matrice de détection unique.

**14.** Procédé d'imagerie optique utilisant un appareil une quelconque revendication précédente, comprenant les étapes consistant à :

recevoir une lumière de bras de référence à partir du bras de référence de l'interféromètre et une lumière de d'échantillon à partir du bras d'échantillon de l'interféromètre, introduire un retard dans la lumière de bras de référence, et
combiner la lumière de bras de référence retardée et la lumière de bras d'échantillon diviser la lumière combinée en une pluralité de bandes spectrales ;
avec chacun des détecteurs, détecter au moins une partie des bandes spectrales ;
générer des signaux en fonction des bandes spectrales détestées ; et
traiter les signaux, et déterminer un profil de profondeur de l'échantillon en fonction des signaux.

**15.** Procédé selon la revendication 14, comprenant en outre :

les étapes consistant à détecter une densité spectrale croisée d'une bande respective des spectrales ; et
combiner la densité spectrale croisée détectée par chacun des détecteurs afin de déterminer une densité spectrale complète.

**16.** Procédé selon la revendication 15, comprenant :

l'étape consistant à déterminer le profil de profondeur en fonction de la densité spectrale croisée complète déterminée.

# FIG. 1

FIG. 2

FIG. 3

EP 1 470 410 B1

# FIG. 4

Phase
Tracker

~108

Low-Coherence
Source

104

Splitting
Unit

106

Sample Arm

130

Tissue

~110

Spectral
Separating
Unit

112

$\Delta\lambda_0$ ... $\Delta\lambda_{N/2}$ ... $\Delta\lambda_N$

114

CCD Detector Array 1

115

CCD Detector Array 2

Processing
and
Display Unit

122

FIG. 5

FIG. 6

FIG. 7

FIG. 8

Sample path fiber          Reference path fiber

402          400

Grating          404

Wavelength

Array of Detectors

406

Interference
Fringes

FIG. 9

700

Balanced detection outputs to
spectral detection units

702 — Source

splitter

706

To sample
probe

splitter 714

splitter

704

splitter

splitter 716 712

708

710 PM

RSOD

722

D1

726

Modulation
Signal

Mixer

D2

Error signal

720

724

Processing unit, sum
generation of offset and
modulation signal

730

Phase
Modulator
Driver

732

728

FIG. 10

32

$\lambda_1$ $\lambda_2$ $\lambda_3$ $\lambda_4$ $\lambda_5$ $\lambda_6$ $\lambda_N$

D1 D2 D3 D4 D5 D6 ········ DN

BPF BPF BPF BPF BPF BPF BPF

A/D ▸ Storage

Reconstruct R(t) from
$B_1(t)$ ...... $B_N(t)$

OR

FFT

FFT

Add FFT's

Reconstructed LCI or OCT
Signal

Further Processing for
Polarization/Spectroscopy
/Doppler images

Display and/or Storage

FIG. 11

# FIG. 12

502

Source 1

504

Source 2

Source 3

506

80/20
Splitter

50/50
Splitter

508

To Slitlamp

510

50/50
Splitter

50/50
splitter

AOM 1    AOM 2

Balanced
detection
outputs

114

115

FIG. 13

FIG. 14

600

620
Sample arm

616

Source

Fiber
Splitter

622
Reference arm

618

602  PM

Detector    606

RSOD

604

608

610

Mixer

Modulation
Signal

Phase
Modulator
Driver

Error signal

Processing unit, generating sum
of offset and modulation signal

612

614

FIG. 15

FIG.15A

FIG.15B

```
        ┌──────────────┐
        │   MEASURE    │◄────────────────────────┐
        │  SIGNAL X₁   │                          │
        └──────┬───────┘                          │
               │                                  │
        ┌──────▼───────┐                          │
        │  INCREASE φ  │                          │
        └──────┬───────┘                          │
               │                                  │
        ┌──────▼───────┐                          │
        │   MEASURE    │                          │
        │  SIGNAL X₂   │                          │
        └──────┬───────┘                          │
               │                                  │
        ┌──────▼───────┐                          │
        │  CALCULATE   │                          │
        │  (X₂-X₁)     │                          │
        └──────┬───────┘                          │
               │                                  │
          ◇────▼────◇                   ┌─────────────────┐
         ╱ IS A(X₂-X₁) ╲     NO         │  CHANGE φ  BY   │
        ◇ WITHIN RANGE   ◇─────────────►│  A(X₂-X₁)-m2π   │
        ╲  FOR PHASE    ╱               └─────────────────┘
         ╲ MODULATION? ╱
          ◇────┬────◇
               │ YES
        ┌──────▼───────┐
        │  CHANGE φ    │
        │  BY A(X₂-X₁) │
        └──────────────┘
```

FIG. 15C

40

FIG. 16

FIG. 17

# FIG. 18

FIG. 19

# FIG. 20

## FIG. 21

**EP 1 470 410 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5321501 A
- US 6134003 A
- US 5317389 A
- US 13681302 A